# EUROPÄISCHE PATENTSCHRIFT

(11) **EP 1 123 386 B1**
(45) Veröffentlichungstag und Bekanntmachung des Hinweises auf die Patenterteilung: **27.12.2006**
(21) Anmeldenummer: 99952558.7
(22) Anmeldetag: 13.10.1999
(51) Int. Cl.: C12N 9/78, C12N 15/63, C12P 41/00, C12P 7/42, C12R 1/05

(54) **VERFAHREN ZUR HERSTELLUNG CHIRALER CARBONSÄUREN AUS NITRILEN MIT HILFE EINER NITRILASE ODER MIKROORGANISMEN, DIE EIN GEN FÜR DIE NITRILASE ENTHALTEN**
METHOD FOR PRODUCING CHIRAL CARBOXYLIC ACIDS FROM NITRILES WITH THE ASSISTANCE OF A NITRILASE OR MICROORGANISMS WHICH CONTAIN A GENE FOR THE NITRILASE
PROCEDE DE PRODUCTION D'ACIDES CARBOXYLIQUES CHIRAUX A PARTIR DE NITRILES, A L'AIDE D'UNE NITRILASE OU DE MICRO-ORGANISMES COMPORTANT UN GENE CODANT POUR LA NITRILASE

(30) Priorität: 19.10.1998 DE 19848129
(43) Veröffentlichungstag der Anmeldung: 16.08.2001
(73) Patentinhaber: BASF Aktiengesellschaft, 67056 Ludwigshafen (DE)
(72) Erfinder: RESS-LÖSCHKE, Marion, D-69221 Dossenheim (DE); FRIEDRICH, Thomas, D-64283 Darmstadt (DE); HAUER, Bernhard, D-67136 Fussgönheim (DE); MATTES, Ralf, D-70619 Stuttgart (DE); ENGELS, Dirk, D-72141 Walddorfhäslach (DE)
(86) Internationale Anmeldenummer: PCT/EP1999/007679
(87) Internationale Veröffentlichungsnummer: WO 2000/023577

(56) Entgegenhaltungen:
- EP-A- 0 348 901
- EP-A- 0 449 648
- KOBAYASHI M ET AL: "NITRILASE IN BIOSYNTHESIS OF THE PLANT HORMONE INDOLE-3-ACETIC ACID FROM INDOLE-3-ACETONITRILE: CLONING OF THE ALCALIGENES GENE AND SITE-DIRECTED MUTAGENESIS OF CYSTEINE RESIDUES" PROCEEDINGS OF THE NATIONAL ACADEMY OF SCIENCES OF USA,US,NATIONAL ACADEMY OF SCIENCE. WASHINGTON, Bd. 90, 1. Januar 1993 (1993-01-01), Seiten 247-251, XP002036846 ISSN: 0027-8424
- PATENT ABSTRACTS OF JAPAN vol. 00, no. 18471, 2. April 1994 (1994-04-02) & JP 06 153968 A (NITTO CHEM.IND. LTD), 3. Juni 1994 (1994-06-03)
- NAGASAWA, T. ET AL.: "A novel nitrilase, arylacetonitrilase, of Alcaligenes faecalis JM3" EUROPEAN JOURNAL OF BIOCHEMISTRY, Bd. 194, 1990, Seiten 765-772, XP000881330 in der Anmeldung erwähnt
- YAMAMOTO, K. ET AL.: "Purification and characterization of the nitrilase from Alcaligenes faecalis ATCC8750 responsible for enantioselective hydrolysis of mandelonitrile" JOURNAL OF FERMENTATION AND BIOENGINEERING, Bd. 73, Nr. 6, 1992, Seiten 425-430, XP002132430

### SEQUENZPROTOKOLL

(1) ALGEMEINE INFORMATION:
   (i) ANMELDER:
      (A) NAME: BASF Aktiengesellschaft
      (B) STRASSE: Carl-Bosch-Strasse 38
      (C) ORT: Ludwigshafen
      (D) BUNDESLAND: Rheinland-Pfalz
      (E) LAND: Bundesrepublik Deutschland
      (F) POSTLEITZAHL: D-67056
   (ii) ANMELDETITEL: Verfahren zur Herstellung chiraler Carbonsaeuren aus Nitrilen mit Hilfe einer Nitrilase oder Mikroorganismen, die ein Gen fuer die Nitrilase enthalten
   (iii) ANZAHL DER SEQUENZEN: 9
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 1071 Basenpaare
      (B) ART: Nukleinsäure
      (C) STRANGFORM: Doppel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Alcaligenes faecalis
      (B) STAMM: 1650
   (ix) MERKMALE:
      (A) NAME/SCHLÜSSEL: CDS
      (B) LAGE: 1..1071
   (xi) SEQUENZBESCHREIBUNG: SEQ ID NO: 1:
   ATG CAG ACA AGA AAA ATC GTC CGG GCA GCC GCC GTA CAG GCC GCC TCT        48
   Met Gln Thr Arg Lys Ile Val Arg Ala Ala Ala Val Gln Ala Ala Ser
     1               5                  10                  15
   CCC AAC TAC GAT CTG GCA ACG GGT GTT GAT AAA ACC ATT GAG CTG GCT         96
   Pro Asn Tyr Asp Leu Ala Thr Gly Val Asp Lys Thr Ile Glu Leu Ala
                   20                  25                  30
   CGT CAG GCC CGC GAT GAG GGC TGT GAC CTG ATC GTG TTT GGT GAA ACC        144
   Arg Gln Ala Arg Asp Glu Gly Cys Asp Leu Ile Val Phe Gly Glu Thr
               35                  40                  45
   TGG CTG CCC GGA TAT CCC TTC CAC GTC TGG CTG GGC GCA CCG GCC TGG        192
   Trp Leu Pro Gly Tyr Pro Phe His Val Trp Leu Gly Ala Pro Ala Trp
           50                  55                  60
   TCG CTG AAA TAC AGT GCC CGC TAC TAT GCC AAC TCG CTC TCG CTG GAC        240
   Ser Leu Lys Tyr Ser Ala Arg Tyr Tyr Ala Asn Ser Leu Ser Leu Asp
    65                  70                  75                  80
   AGT GCA GAG TTT CAA CGC ATT GCC CAG GCC GCA CGG ACC TTG GGT ATT        288
   Ser Ala Glu Phe Gln Arg Ile Ala Gln Ala Ala Arg Thr Leu Gly Ile
                       85                  90                  95
   TTC ATC GCA CTG GGT TAT AGC GAG CGC AGC GGC GGC AGC CTT TAC CTG        336
   Phe Ile Ala Leu Gly Tyr Ser Glu Arg Ser Gly Gly Ser Leu Tyr Leu
                   100                 105                 110
   GGC CAA TGC CTG ATC GAC GAC AAG GGC GAG ATG CTG TGG TCG CGT CGC        384
   Gly Gln Cys Leu Ile Asp Asp Lys Gly Glu Met Leu Trp Ser Arg Arg
               115                 120                 125
   AAA CTC AAA CCC ACG CAT GTA GAG CGC ACC GTA TTT GGT GAA GGT TAT        432
   Lys Leu Lys Pro Thr His Val Glu Arg Thr Val Phe Gly Glu Gly Tyr
           130                 135                 140
   GCC CGT GAT CTG ATT GTG TCC GAC ACA GAA CTG GGA CGC GTC GGT GCT        480
   Ala Arg Asp Leu Ile Val Ser Asp Thr Glu Leu Gly Arg Val Gly Ala
   145                 150                 155                 160
   CTA TGC TGC TGG GAG CAT TTG TCG CCC TTG AGC AAG TAC GCG CTG TAC        528
   Leu Cys Cys Trp Glu His Leu Ser Pro Leu Ser Lys Tyr Ala Leu Tyr
                       165                 170                 175
   TCC CAG CAT GAA GCC ATT CAC ATT GCT GCC TGG CCG TCG TTT TCG CTA        576
   Ser Gln His Glu Ala Ile His Ile Ala Ala Trp Pro Ser Phe Ser Leu
                   180                 185                 190
   TAC AGC GAA CAG GCC CAC GCC CTC AGT GCC AAG GTG AAC ATG GCT GCC        624
   Tyr Ser Glu Gln Ala His Ala Leu Ser Ala Lys Val Asn Met Ala Ala
               195                 200                 205
   TCG CAA ATC TAT TCG GTT GAA GGC CAG TGC TTT ACC ATC GCC GCC AGC        672
   Ser Gln Ile Tyr Ser Val Glu Gly Gln Cys Phe Thr Ile Ala Ala Ser
           210                 215                 220
   AGT GTG GTC ACC CAA GAG ACG CTA GAC ATG CTG GAA GTG GGT GAA CAC      720
   Ser Val Val Thr Gln Glu Thr Leu Asp Met Leu Glu Val Gly Glu His
   225                 230                 235                 240
   AAC GCC CCC TTG CTG AAA GTG GGC GGC GGC AGT TCC ATG ATT TTT GCG      768
   Asn Ala Pro Leu Leu Lys Val Gly Gly Gly Ser Ser Met Ile Phe Ala
                   245                 250                 255
   CCG GAC GGA CGC ACA CTG GCT CCC TAC CTG CCT CAC GAT GCC GAG GGC      816
   Pro Asp Gly Arg Thr Leu Ala Pro Tyr Leu Pro His Asp Ala Glu Gly
               260                 265                 270
   TTG ATC ATT GCC GAT CTG AAT ATG GAG GAG ATT GCC TTC GCC AAA GCG      864
   Leu Ile Ile Ala Asp Leu Asn Met Glu Glu Ile Ala Phe Ala Lys Ala
           275                 280                 285
   ATC AAT GAC CCC GTA GGC CAC TAT TCC AAA CCC GAG GCC ACC CGT CTG      912
   Ile Asn Asp Pro Val Gly His Tyr Ser Lys Pro Glu Ala Thr Arg Leu
       290                 295                 300
   GTG CTG GAC TTG GGG CAC CGA GAC CCC ATG ACT CGG GTG CAC TCC AAA      960
   Val Leu Asp Leu Gly His Arg Asp Pro Met Thr Arg Val His Ser Lys
   305                 310                 315                 320
   AGC GTG ACC AGG GAA GAG GCT CCC GAG CAA GGT GTG CAA AGC AAG ATT     1008
   Ser Val Thr Arg Glu Glu Ala Pro Glu Gln Gly Val Gln Ser Lys Ile
                   325                 330                 335
   GCC TCA GTC GCT ATC AGC CAT CCA CAG GAC TCG GAC ACA CTG CTA GTG     1056
   Ala Ser Val Ala Ile Ser His Pro Gln Asp Ser Asp Thr Leu Leu Val
               340                 345                 350
   CAA GAG CCG TCT TGA                                                 1071
   Gln Glu Pro Ser
           355
(2) INFORMATION ZU SEQ ID NO: 2:
   (i) SEQUENZ CHARAKTERISTIKA:
      (A) LÄNGE: 356 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Gln Thr Arg Lys Ile Val Arg Ala Ala Ala Val Gln Ala Ala Ser
     1               5                  10                  15
   Pro Asn Tyr Asp Leu Ala Thr Gly Val Asp Lys Thr Ile Glu Leu Ala
                20                  25                  30
   Arg Gln Ala Arg Asp Glu Gly Cys Asp Leu Ile Val Phe Gly Glu Thr
           35                  40                  45
   Trp Leu Pro Gly Tyr Pro Phe His Val Trp Leu Gly Ala Pro Ala Trp
       50                  55                  60
   Ser Leu Lys Tyr Ser Ala Arg Tyr Tyr Ala Asn Ser Leu Ser Leu Asp
   65                  70                  75                  80
   Ser Ala Glu Phe Gln Arg Ile Ala Gln Ala Ala Arg Thr Leu Gly Ile
                   85                  90                  95
   Phe Ile Ala Leu Gly Tyr Ser Glu Arg Ser Gly Gly Ser Leu Tyr Leu
               100                 105                 110
   Gly Gln Cys Leu Ile Asp Asp Lys Gly Glu Met Leu Trp Ser Arg Arg
           115                 120                 125
   Lys Leu Lys Pro Thr His Val Glu Arg Thr Val Phe Gly Glu Gly Tyr
       130                 135                 140
   Ala Arg Asp Leu Ile Val Ser Asp Thr Glu Leu Gly Arg Val Gly Ala
   145                 150                 155                 160
   Leu Cys Cys Trp Glu His Leu Ser Pro Leu Ser Lys Tyr Ala Leu Tyr
                   165                 170                 175
   Ser Gln His Glu Ala Ile His Ile Ala Ala Trp Pro Ser Phe Ser Leu
               180                 185                 190
   Tyr Ser Glu Gln Ala His Ala Leu Ser Ala Lys Val Asn Met Ala Ala
           195                 200                 205
   Ser Gln Ile Tyr Ser Val Glu Gly Gln Cys Phe Thr Ile Ala Ala Ser
       210                 215                 220
   Ser Val Val Thr Gln Glu Thr Leu Asp Met Leu Glu Val Gly Glu His
   225                 230                 235                 240
   Asn Ala Pro Leu Leu Lys Val Gly Gly Gly Ser Ser Met Ile Phe Ala
                   245                 250                 255
   Pro Asp Gly Arg Thr Leu Ala Pro Tyr Leu Pro His Asp Ala Glu Gly
               260                 265                 270
   Leu Ile Ile Ala Asp Leu Asn Met Glu Glu Ile Ala Phe Ala Lys Ala
           275                 280                 285
   Ile Asn Asp Pro Val Gly His Tyr Ser Lys Pro Glu Ala Thr Arg Leu
       290                 295                 300
   Val Leu Asp Leu Gly His Arg Asp Pro Met Thr Arg Val His Ser Lys
   305                 310                 315                 320
   Ser Val Thr Arg Glu Glu Ala Pro Glu Gln Gly Val Gln Ser Lys Ile
   325 330 335
   Ala Ser Val Ala Ile Ser His Pro Gln Asp Ser Asp Thr Leu Leu Val
   340 345 350
   Gln Glu Pro Ser
   355
(2) INFORMATION ZU SEQ ID NO: 3:
   (i) SEQUENZ CHARAKTERISTIKA:
      (A) LÄNGE: 39 Aminosäuren
      (B) ART: Aminosäure
      (D) TOPOLOGIE: linear
   (ii) ART DES MOLEKÜLS: Peptid
   (iii) HYPOTHETISCH: NEIN
   (iii) ANTISENSE: NEIN
   (v) ART DES FRAGMENTS: N-Terminus
   (vi) URSPRÜNLICHE HERKUNFT:
      (A) ORGANISMUS: Alcaligenes faecalis
      (B) STAMM: 1650
   (vii) UNMITTELBARE HERKUNFT:
      (B) CLON: Nitrilase
   (xi) SEQUENZBESCHREIBUNG: SEQ ID NO: 3:
   Met Gln Thr Arg Lys Ile Val Arg Ala Ala Ala Val Gln Ala Ala Ser
   1 5 10 15
   Pro Asn Tyr Asp Leu Ala Thr Gly Val Asp Lys Thr Ile Glu Leu Ala
   20 25 30
   Arg Gln Ala Arg Asp Glu Gly
   35
(2) INFORMATION ZU SEQ ID NO: 4:
   (i) SEQUENZ CHARAKTERISTIKA:
      (A) LÄNGE: 21 Aminosäuren
      (B) ART: Aminosäure
      (D) TOPOLOGIE: linear
   (ii) ART DES MOLEKÜLS: Peptid
   (iii) HYPOTHETISCH: NEIN
   (iii) ANTISENSE: NEIN
   (v) ART DES FRAGMENTS: inneres
   (vi) URSPRÜNLICHE HERKUNFT:
      (A) ORGANISMUS: Alcaligenes faecalis
      (B) STAMM: 1650
   (vii) UNMITTELBARE HERKUNFT:
      (B) CLON: Nitrilase
   (xi) SEQUENZBESCHREIBUNG: SEQ ID NO: 4:
   Glu Glu Ala Pro Glu Gln Gly Val Gln Ser Lys Ile Ala Ser Val Ala
   1               5                   10                  15
   Ile Ser His Pro Gln
               20
(2) INFORMATION ZU SEQ ID NO: 5:
   (i) SEQUENZ CHARAKTERISTIKA:
      (A) LÄNGE: 11 Aminosäuren
      (B) ART: Aminosäure
      (D) TOPOLOGIE: linear
   (ii) ART DES MOLEKÜLS: Peptid
   (iii) HYPOTHETISCH: NEIN
   (iii) ANTISENSE: NEIN
   (v) ART DES FRAGMENTS: inneres
   (vi) URSPRÜNLICHE HERKUNFT:
      (A) ORGANISMUS: Alcaligenes faecalis
      (B) STAMM: 1650
   (vii) UNMITTELBARE HERKUNFT:
      (B) CLON: Nitrilase
   (xi) SEQUENZBESCHREIBUNG: SEQ ID NO: 5:
   Glu Glu Ala Pro Glu Gln Gly Val Gln Ser Lys
   1               5                   10
(2) INFORMATION ZU SEQ ID NO: 6:
   (i) SEQUENZ CHARAKTERISTIKA:
      (A) LÄNGE: 23 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Alcaligenes faecalis
      (B) STAMM: 1650
   (vii) UNMITTELBARE HERKUNFT:
      (B) CLON: Nitrilase
   (xi) SEQUENZBESCHREIBUNG: SEQ ID NO: 6:
      ATGCAGACNA GNAARATCGT SCG 23
(2) INFORMATION ZU SEQ ID NO: 7:
   (i) SEQUENZ CHARAKTERISTIKA:
      (A) LÄNGE: 20 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Alcaligenes faecalis
      (B) STAMM: 1650
   (vii) UNMITTELBARE HERKUNFT:
      (B) CLON: Nitrilase
   (xi) SEQUENZBESCHREIBUNG: SEQ ID NO: 7:
      TNGCSACNGA NGCRATCTTG 20
(2) INFORMATION ZU SEQ ID NO: 8:
   (i) SEQUENZ CHARAKTERISTIKA:
      (A) LÄNGE: 31 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Alcaligenes faecalis
      (B) STAMM: 1650
   (vii) UNMITTELBARE HERKUNFT:
      (B) CLON: Nitrilase
   (xi) SEQUENZBESCHREIBUNG: SEQ ID NO: 8:
      TTAATCATAT GCAGACAAGA AAAATCGTCC G 31
(2) INFORMATION ZU SEQ ID NO: 9:
   (i) SEQUENZ CHARAKTERISTIKA:
      (A) LÄNGE: 32 Basenpaare
      (B) ART: Nukleinsäure
      (C) STRANGFORM: Einzel
      (D) TOPOLOGIE: linear
   (ii) ART DES MOLEKÜLS: DNS (genomisch)
   (iii) HYPOTHETISCH: NEIN
   (iii) ANTISENSE: NEIN
   (vi) URSPRÜNLICHE HERKUNFT:
      (A) ORGANISMUS: Alcaligenes faecalis
      (B) STAMM: 1650
   (vii) UNMITTELBARE HERKUNFT:
      (B) CLON: Nitrilase
   (xi) SEQUENZBESCHREIBUNG: SEQ ID NO: 9:
      AAGGATCCTC AAGACGGCTC TTGCACTAGC AG 32

## Beschreibung

Die Erfindung betrifft Nukleinsäuresequenzen, die für ein Polypeptid mit Nitrilaseaktivität codieren, Nukleinsäurekonstrukte enthaltend die Nukleinsäuresequenzen sowie Vectoren enthaltend die Nukleinsäuresequenzen oder die Nukleinsäurekonstrukte. Die Erfindung betrifft weiterhin Aminosäuresequenzen, die durch die Nukleinsäuresequenzen codiert werden und Mikroorganismen enthaltend die Nukleinsäuresequenzen, die Nukleinsäurekonstrukte oder Vectoren enthaltend die Nukleinsäuresequenzen oder die Nukleinsäurekonstrukte.

Außerdem betrifft die Erfindung ein Verfahren zur Herstellung chiraler Carbonsäuren aus den racemischen Nitrilen.

Chirale Carbonsäuren sind gesuchte Verbindungen für die organischen Synthesechemie. Sie sind Ausgangsprodukte für eine Vielzahl von pharmazeutischen Wirkstoffen oder Wirkstoffen für den Pflanzenschutz. Chirale Carbonsäuren können zur klassischen Racematspaltung über Diastereomeresalze verwendet werden. So wird R-(-)- oder S-(-)-Mandelsäure beispielsweise zur Racematspaltung racemischer Amine eingesetzt. R-(-)-Mandelsäure wird außerdem als Zwischenprodukt bei der Synthese halbsynthetischer Antibiotika und einer Vielzahl landwirtschaftlicher Produkte genutzt wird.

Aus der Literatur sind eine Reihe verschiedener Synthesezugänge zu chiralen Carbonsäuren bekannt. So werden beispielsweise optisch aktive Aminosäuren technisch über fermentative Verfahren gewonnen. Von Nachteil dabei ist, daß für jede Aminosäure ein eigenes Verfahren entwickelt werden muß. Um eine möglichst breite Palette verschiedener Verbindungen herstellen zu können, werden deshalb chemische oder enzymatische Verfahren verwendet. Nachteilig bei den chemischen Verfahren ist, daß das Stereozentrum in der Regel in mehrstufigen, nicht breit anwendbaren Synthese umständlich aufgebaut werden muß.

Die enzymatische Synthese chiraler Carbonsäuren sind einer Reihe von Patenten oder Patentanmeldungen zu entnehmen. WO92/05275 beschreibt die Synthese enantiomerer α-Hydroxy-α-alkyl- oder α-Alkylcarbonsäuren in Gegenwart biologischen Materials. In EP-B-0 348 901 wird ein Verfahren zur Herstellung von optisch aktiven α-substituierten organischen Säuren mit Mikroorganismen der Gattungen Alcaligenes, Pseudomonas, Rhodopseudomonas, Corynebacterium sp. Stamm KO-2-4, Acinetobacter, Bacillus, Mycobacterium, Rhodococcus und Candida beansprucht. Die Herstellung von L-α-Aminosäuren wird mit Mikroorganismen wird in EP-B-0 332 379 beansprucht.

Die Herstellung von α-Hydroxycarbonsäuren speziell die Herstellung von optisch aktiver Milchsäure oder Mandelsäure mit verschiedenen Mikroorganismen wie Mikroorgansimen der Gattungen Alcaligenes, Aureobacterium, Pseudomonas, Rhodopseudomonas, Corynebacterium, Acinetobacter, Caseobacter, Bacillus, Mycobacterium, Rhodococcus, Brevibacterium, Nocardia, Variovorax, Arthrobacter und Candida oder mit Enzymen wird in den Schutzrechten EP-A-0 348 901 oder seinem US-Äquivalent US 5,283,193, EP-A-0 449 648, EP-B-0 473 328, EP-B-0 527 553 oder seinem US-Äquivalent US 5,296,373, EP-A-0 610 048, EP-A-0 610 049, EP-A-0 666 320 oder WO97/32030 beschrieben.

Von Nachteil bei diesen Prozessen ist, daß sie häufig nur zu Produkten mit einer geringen optischen Reinheit führen und/oder daß sie nur mit geringen Raum-Zeit-Ausbeuten ablaufen. Dies führt zu wirtschaftlich unattraktiven Prozessen. Auch der Versuch durch Zugabe von Substanzen wie Sulfit, Disulfit, Dithionit, Hypophosphit oder Phosphit die Produktivität zu erhöhen (siehe EP-A-0 486 289) oder über die Verwendung von Mikroorganismen, die eine erhöhte Resistenz gegenüber α-Hydroxynitrilen aufweisen (siehe WO97/32030), führt zu keiner nennenswerten Steigerung der Produktivität.

Es war daher die Aufgabe der Erfindung ein leichtes, kostengünstiges, breit anwendbares Verfahren zur Herstellung von optisch aktiven, chiralen Carbonsäuren zu entwickeln, das die oben genannten Nachteile nicht aufweist.

Diese Aufgabe wurde durch das erfindungsgemäße Verfahren zur Herstellung von chiralen Carbonsäuren der allgemeinen Formel I 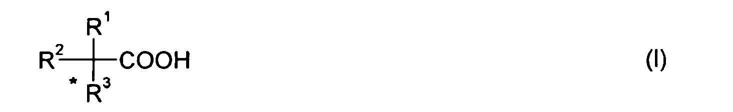 dadurch gekennzeichnet, daß man racemische Nitrile der allgemeinen Formel II 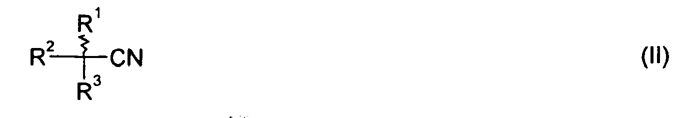 in Gegenwart einer Aminosäuresequenz, die codiert wird durch eine Nukleinsäuresequenz ausgewählt aus der Gruppe
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 1 dargestellten Nukleinsäuresequenz ableiten,
c) Homologe der in SEQ ID NO: 1 dargestellten Nukleinsäuresequenz, die für Polypeptide Kodieren die mindestens 98% Homologie über den gesamten Sequenzbereich zu Seq ID NO: 2 aufweisen ohne daß die enzymatische Wirkung der Polypeptide reduziert ist,
oder einem wachsenden, ruhenden oder aufgeschlossenen Mikroorganismus, der entweder eine Nukleinsäuresequenz aus der oben genannten Gruppe oder ein Nukleinsäurekonstrukt, das eine Nukleinsäure aus der genannten Gruppe mit einem oder mehreren Regulationssignalen verknüpft, enthält, umsetzt und wobei mindestens 25 mmol Nitril/h pro mg Protein oder 25 mmol Nitril/h pro g Trockengewicht zu den chiralen Carbonsäuren umgesetzt werden,
wobei die Substituenten und Variablen in den Formeln I und II folgende Bedeutung haben:
* ein optisch aktives Zentrum
- R¹,R², R³: unabhängig voneinander Wasserstoff, substituiertes oder unsubstituiertes, verzweigtes oder unverzweigtes C₁-C₁₀-Alkyl-, C₂-C₁₀-Alkenyl-, substituiertes oder unsubstituiertes Aryl-, Hetaryl-, OR⁴ oder NR⁴R⁵ und wobei die Reste R¹, R² und R³ immer unterschiedlich sind,
- R⁴: Wasserstoff, substituiertes oder unsubstituiertes, verzweigtes oder unverzweigtes C₁-C₁₀-Alkyl-, C₂-C₁₀-Alkenyl-, C₁-C₁₀-Alkylcarbonyl-, C₂-C₁₀-Alkenylcarbonyl-, Aryl-, Aryl-carbonyl-, Hetaryl- oder Hetarylcarbonyl-,
- R⁵: Wasserstoff, substituiertes oder unsubstituiertes, verzweigtes oder unverzweigtes C₁-C₁₀-Alkyl-, C₂-C₁₀-Alkenyl-, Aryl-oder Hetaryl-, gelöst.

R¹, R², R³ bezeichnen in den Verbindungen der Formeln I und II unabhängig voneinander Wasserstoff, substituiertes oder unsubstituiertes, verzweigtes oder unverzweigtes C₁-C₁₀-Alkyl-, C₂-C₁₀-Alkenyl-, substituiertes oder unsubstituiertes Aryl-, Hetaryl-, OR⁴ oder NR⁴R⁵ und wobei die Reste R¹, R² und R³ immer unterschiedlich sind

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Bevorzugt sind Methyl, Ethyl, n-Propyl, n-Butyl, i-Propyl oder i-Butyl.

Als Alkenylreste seien verzweigte oder unverzweigte C₂-C₁₀-Alkenylketten wie beispielsweise Ethenyl, Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, Nonenyl oder Dekenyl genannt. Bevorzugt sind Ethenyl, Propenyl, Butenyl oder Pentenyl.

Als Aryl- seien substituiertes und unsubstituiertes Arylreste, die 6 bis 20 Kohlenstoffatome im Ring oder Ringsystem enthalten genannt. Dabei kann es sich um aneinander kondensierte aromatische Ringe handeln oder um aromatische Ringe, die über Alkyl-, Alkylcarbonyl-, Alkenyl- oder Alkenylcarbonylketten, Carbonyl, Sauerstoff oder Stickstoff verbrückt sind. Die Arylreste können gegebenenfalls noch über eine C₁-C₁₀-Alkyl-, C₃-C₈-Alkenyl-, C₃-C₆-Alkinyl- oder C₃-C₈-Cycloalkylkette an das Grundgerüst gebunden sein. Bevorzugt sind Phenyl oder Naphtyl.

Als Hetaryl- seien substituierte oder unsubstituierte, einfache oder kondensierte aromatische Ringsysteme mit einem oder mehreren heteroaromatischen 3- bis 7gliedrigen Ringen, die ein oder mehrere Heteroatome wie N, O oder S enthalten können und gegebenenfalls über eine C₁-C₁₀-Alkyl-, C₃-C₈-Alkenyl- oder C₃-C₈-Cycloalkylkette an das Grundgerüst gebunden sein können, genannt. Beispiele für derartige Hetarylreste sind Pyrazol, Imidazol, Oxazol, Isooxazol, Thiazol, Triazol, Pyridin, Chinolin, Isochinolin, Acridin, Pyrimidin, Pyridazin, Pyrazin, Phenazin, Purin oder Pteridin. Die Hetarylreste können über die Heteroatome oder über die verschiedenen Kohlenstoffatome im Ring oder Ring system oder über die Substituenten an das Grundgerüst gebunden sein. Bevorzugt sind Pyridin, Imidazol, Pyrimidin, Purin, Pyrazin oder Chinolin.

Als Substituenten der genannten Reste von R¹. R² oder R³ kommen beispielsweise ein oder mehrere Substituenten wie Halogen wie Fluor, Chlor oder Brom, Thio, Nitro, Amino, Hydroxy, Alkyl, Alkoxy, Alkenyl, Alkenyloxy, Alkinyl oder weitere aromatischen oder weitere gesättigte oder ungesättigte nicht aromatische Ringen oder Ringsystemen in Frage. Bevorzugt sind Alkylreste wie C₁-C₆-Alkyl wie Methyl, Ethyl, Propyl oder Butyl, Aryl wie Phenyl, Halogen wie Chlor, Fluor oder Brom, Hydroxy oder Amino.

R⁴ bezeichnet in den Resten OR⁴ oder NR⁴R⁵ Wasserstoff, substituiertes oder unsubstituiertes, verzweigtes oder unverzweigtes C₁-C₁₀-Alkyl-, C₂-C₁₀-Alkenyl-, C₁-C₁₀-Alkylcarbonyl-, C₂-C₁₀-Alkenylcarbonyl-, Aryl-, Arylcarbonyl-, Hetaryl- oder Hetarylcarbonyl.

Als Alkylreste seien substituierte oder unsubstituierte verzweigte oder unverzweigte C₁-C₁₀-Alkylketten wie beispielsweise Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, n-Octyl, n-Nonyl oder n-Decyl genannt. Bevorzugt sind Methyl, Ethyl, n-Propyl, n-Butyl, i-Propyl oder i-Butyl.

Als Alkenylreste seien verzweigte oder unverzweigte C₂-C₁₀-Alkenylketten wie beispielsweise Ethenyl, Propenyl, 1-Butenyl, 2-Butenyl, 3-Butenyl, 2-Methylpropenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl, 1-Heptenyl, 2-Heptenyl, 3-Heptenyl, 4-Heptenyl, 5-Heptenyl, 6-Heptenyl, 1-Octenyl, 2-Octenyl, 3-Octenyl, 4-Octenyl, 5-Octenyl, 6-Octenyl, 7-Octenyl, Nonenyl oder Dekenyl genannt. Bevorzugt sind Ethenyl, Propenyl, Butenyl oder Pentenyl.

Als Alkylcarbonylreste seien substituierte oder unsubstituierte verzweigte oder unverzweigte C₁-C₁₀-Alkylcarbonylketten wie beispielsweise Methylcarbonyl, Ethylcarbonyl, n-Propylcarbonyl, 1-Methylethylcarbonyl, n-Butylcarbonyl, 1-Methyl-propylcarbonyl-, 2-Methylpropylcarbonyl, 1,1-Dimethylethylcarbonyl, n-Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, n-Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl, 1-Ethyl-2-methylpropylcarbonyl, n-Heptylcarbonyl, n-Octylcarbonyl, n-Nonylcarbonyl oder n-Decylcarbonyl genannt. Bevorzugt sind Methylcarbonyl, Ethylcarbonyl, n-Propylcarbonyl, n-Butylcarbonyl, i-Propylcarbonyl oder i-Butylcarbonyl.

Als Alkenylcarbonylreste seien verzweigte oder unverzweigte C₂-C₁₀-Alkenylcarbonylketten wie beispielsweise Ethenylcarbonyl, Propenylcarbonyl, 1-Butenylcarbonyl, 2-Butenylcarbonyl, 3-Butenylcarbonyl, 2-Methylpropenylcarbonyl, 1-Pentenylcarbonyl, 2-Pentenylcarbonyl, 3-Pentenylcarbonyl, 4-Pentenylcarbonyl, 1-Methyl-1-butenylcarbonyl, 2-Methyl-1-butenylcarbonyl, 3-Methyl-1-butenylcarbonyl, 1-Methyl-2-butenylcarbonyl, 2-Methyl-2-butenylcarbonyl, 3-Methyl-2-butenylcarbonyl, 1-Methyl-3-butenylcarbonyl, 2-Methyl-3-butenylcarbonyl, 3-Methyl-3-butenylcarbonyl, 1,1-Dimethyl-2-propenylcarbonyl, 1,2-Dimethyl-1-propenylcarbonyl, 1,2-Dimethyl-2-propenylcarbonyl, 1-Ethyl-1-propenylcarbonyl, 1-Ethyl-2-propenylcarbonyl, 1-Hexenylcarbonyl, 2-Hexenylcarbonyl, 3-Hexenylcarbonyl, 4-Hexenylcarbonyl, 5-Hexenylcarbonyl, 1-Methyl-1-pentenylcarbonyl, 2-Methyl-1-pentenylcarbonyl, 3-Methyl-1-pentenylcarbonyl, 4-Methyl-1-pentenylcarbonyl, 1-Methyl-2-pentenylcarbonyl, 2-Methyl-2-pentenylcarbonyl, 3-Methyl-2-pentenylcarbonyl, 4-Methyl-2-pentenylcarbonyl, 1-Methyl-3-pentenylcarbonyl, 2-Methyl-3-pentenylcarbonyl, 3-Methyl-3-pentenylcarbonyl, 4-Methyl-3-pentenylcarbonyl, 1-Methyl-4-pentenylcarbonyl, 2-Methyl-4-pentenylcarbonyl, 3-Methyl-4-pentenylcarbonyl, 4-Methyl-4-pentenylcarbonyl, 1,1-Dimethyl-2-butenylcarbonyl, 1,1-Dimethyl-3-butenylcarbonyl, 1,2-Dimethyl-1-butenylcarbonyl, 1,2-Dimethyl-2-butenylcarbonyl, 1,2-Dimethyl-3-butenylcarbonyl, 1,3-Dimethyl-1-butenylcarbonyl, 1,3-Dimethyl-2-butenylcarbonyl, 1,3-Dimethyl-3-butenylcarbonyl, 2,2-Dimethyl-3-butenylcarbonyl, 2,3-Dimethyl-1-butenylcarbonyl, 2,3-Dimethyl-2-butenylcarbonyl, 2,3-Dimethyl-3-butenylcarbonyl, 3,3-Dimethyl-1-butenylcarbonyl, 3,3-Dimethyl-2-butenylcarbonyl, 1-Ethyl-1-butenylcarbonyl, 1-Ethyl-2-butenylcarbonyl, 1-Ethyl-3-butenylcarbonyl, 2-Ethyl-1-butenylcarbonyl, 2-Ethyl-2-butenylcarbonyl, 2-Ethyl-3-butenylcarbonyl, 1,1,2-Trimethyl-2-propenylcarbonyl, 1-Ethyl-1-methyl-2-propenylcarbonyl, 1-Ethyl-2-methyl-1-propenylcarbonyl, 1-Ethyl-2-methyl-2-propenylcarbonyl, 1-Heptenylcarbonyl, 2-Heptenylcarbonyl, 3-Heptenylcarbonyl, 4-Heptenylcarbonyl, 5-Heptenylcarbonyl, 6-Heptenylcarbonyl, 1-Octenylcarbonyl, 2-Octenylcarbonyl, 3-Octenylcarbonyl, 4-Octenylcarbonyl, 5-Octenylcarbonyl, 6-Octenylcarbonyl, 7-Octenylcarbonyl, Nonenylcarbonyl oder Dekenylcarbonyl genannt. Bevorzugt sind Ethenylcarbonyl, Propenylcarbonyl, Butenylcarbonyl oder Pentenylcarbonyl.

Als Aryl- seien substituiertes und unsubstituiertes Arylreste, die 6 bis 20 Kohlenstoffatome im Ring oder Ringsystem enthalten genannt. Dabei kann es sich um aneinander kondensierte aromatische Ringe handeln oder um aromatische Ringe, die über Alkyl-, Alkylcarbonyl-, Alkenyl- oder Alkenylcarbonylketten, Carbonyl, Sauerstoff oder Stickstoff verbrückt sind. Die Arylreste können gegebenenfalls noch über eine C₁-C₁₀-Alkyl-, C₃-C₈-Alkenyl-, C₃-C₆-Alkinyl- oder C₃-C₈-Cycloalkylkette an das Grundgerüst gebunden sein. Bevorzugt sind Phenyl oder Naphtyl.

Als Arylcarbonyl- seien substituiertes und unsubstituiertes Arylcarbonylreste, die 6 bis 20 Kohlenstoffatome im Ring oder Ringsystem enthalten genannt. Dabei kann es sich um aneinander kondensierte aromatische Ringe handeln oder um aromatische Ringe, die über Alkyl-, Alkylcarbonyl-, Alkenyl- oder Alkenylcarbonylketten, Carbonyl, Sauerstoff oder Stickstoff verbrückt sind. Bevorzugt sind Phenylcarbonyl oder Naphthylcarbonyl.

Als Hetaryl- seien substituierte oder unsubstituierte, einfache oder kondensierte aromatische Ringsysteme mit einem oder mehreren heteroaromatischen 3- bis 7gliedrigen Ringen, die ein oder mehrere Heteroatome wie N, O oder S enthalten können und gegebenenfalls über eine C₁-C₁₀-Alkyl-, C₃-C₈-Alkenyl- oder C₃-C₈-Cycloalkylkette an das Grundgerüst gebunden sein können, genannt. Beispiele für derartige Hetarylreste sind Pyrazol, Imidazol, Oxazol, Isooxazol, Thiazol, Triazol, Pyridin, Chinolin, Isochinolin, Acridin, Pyrimidin, Pyridazin, Pyrazin, Phenazin, Purin oder Pteridin. Die Hetarylreste können über die Heteroatome oder über die verschiedenen Kohlenstoffatome im Ring oder Ringsystem oder über die Substituenten an das Grundgerüst gebunden sein. Unter Hetarylcarbonylresten sind heteroaromatische Reste zu verstehen, die über einen Carbonylrest an das Grundgerüst gebunden sind. Bevorzugt sind Pyridin, Imidazol, Pyrimidin, Purin, Pyrazin oder Chinolin.

Als Substituenten der genannten Reste von R⁴ kommen beispielsweise ein oder mehrere Substituenten wie Halogen wie Fluor, Chlor oder Brom, Thio, Nitro, Amino, Hydroxy, Alkyl, Alkoxy, Alkenyl, Alkenyloxy, Alkinyl oder weitere aromatischen oder weitere gesättigte oder ungesättigte nicht aromatische Ringen oder Ringsystemen in Frage. Bevorzugt sind Alkylreste wie C₁-C₆-Alkyl wie Methyl, Ethyl, Propyl oder Butyl, Halogen wie Chlor, Fluor oder Brom, Hydroxy oder Amino.

Bevorzugt ist für den Rest R⁴ Wasserstoff.

R⁵ bezeichnet im Rest NR⁴R⁵ Wasserstoff, substituiertes oder unsubstituiertes, verzweigtes oder unverzweigtes C₁-C₁₀-Alkyl-, C₂-C₁₀-Alkenyl-, Aryl- oder Hetaryl-, wobei die Alkyl-, Alkenyl-, Aryl- und Hetarylreste die oben genannte Bedeutung haben. Bevorzugt ist Wasserstoff oder C₁-C₁₀-Alkyl- wie Methyl, Ethyl oder Propyl.

Als Substituenten der genannten Reste von R⁵ kommen beispielsweise ein oder mehrere Substituenten wie Halogen wie Fluor, Chlor oder Brom, Thio, Nitro, Amino, Hydroxy, Alkyl, Alkoxy, Alkenyl, Alkenyloxy, Alkinyl oder weitere aromatischen oder weitere gesättigte oder ungesättigte nicht aromatische Ringen oder Ringsystemen in Frage. Bevorzugt sind Alkylreste wie C₁-C₆-Alkyl wie Methyl, Ethyl, Propyl oder Butyl, Aryl wie Phenyl, Halogen wie Chlor, Fluor oder Brom, Hydroxy oder Amino.

Weiter können zwei benachbarte Substituenten R⁴ oder R⁵ zusammen einen weiteren substituierten oder unsubstituierten aromatischen, gesättigten oder teilweise gesättigten Ring mit 5 bis 6 Atomen im Ring bilden, der ein oder mehrere Heteroatome wie O, N oder S enthalten kann.

Vorteilhaft bedeutet einer der Substituenten R¹, R² oder R³ in den Formeln I und II Aryl wie Phenyl. Weiterhin bedeutet einer der Substituenten R¹, R² oder R³ in den Formeln I und II bevorzugt Hydroxy und einer Wasserstoff oder Methyl.

Das erfindungsgemäße Verfahren wird vorteilhaft bei einem pH-Wert von 4 bis 11, bevorzugt von 4 bis 9 durchgeführt.

Weiterhin werden im Verfahren vorteilhaft von 0,01 bis 10 Gew.-% Nitril oder 0,01 bis 10 Gew.-% eines entsprechenden Aldehyds oder Ketons und 0,01 bis 10 Gew.-% Blausäure verwendet. Vorteilhaft wird das Verfahren mit einem Überschuß an Blausäure durchgeführt. Dies führt unter Umständen zu höheren als den angegebenen Blausäureanteilen. Je nach Nitril können unterschiedliche Mengen an Nitril in der Reaktion verwendet werden. Die geringsten Mengen an Nitril werden vorteilhaft bei Nitrilen (Cyanhydrine) verwendet (= Mengen zwischen 0,01 bis 5 Gew.-%), die im Gleichgewicht mit den entsprechenden Aldehyden und Blausäure stehen. Da der Aldehyd für die Mikroorganismen oder Enzyme in der Regel toxisch ist. Nitrile, die leicht flüchtig sind, werden ebenfalls vorteilhaft in Mengen zwischen 0,01 bis 5 Gew.-% eingesetzt. Bei höheren Cyanhydrin- bzw. Nitrilmengen läuft die Reaktion verzögert ab. Bei Nitrilen, die nur geringe oder nahezu keine Lösungsmitteleigenschaften haben oder Nitrilen, die sich nur in sehr geringen Mengen in wäßrigen Medium lösen, können vorteilhaft auch größere als die oben angegebenen Mengen eingesetzt werden. Zur Erhöhung des Umsatzes und der Ausbeute wird die Reaktion vorteilhafterweise unter kontinuierlicher Zugabe des racemischen Nitrils durchgeführt. Das Produkt kann nach Ende der Reaktion isoliert werden oder aber in einem Bypass kontinuierlich entfernt werden.

Unter den oben genannten, entsprechenden Aldehyden oder Ketonen sind Verbindungen zu verstehen, die nach Reaktion zwischen dem Aldehyd oder Keton und Blausäure ggf. unter Säurekatalyse das Nitril bilden. Die Reaktion zwischen Aldehyd und Blausäure führt zu Cyanhydrinen, die den Vorteil haben, daß sie mit Aldehyd und Blausäure im Gleichgewicht liegen. Durch die Gleichgewichtseinstellung des Cyanhydrins ist es möglich mit einem Enzym, das nur ein Enatiomer des Nitrils umsetzt, trotzdem zu 100 % Ausbeute in der Theorie zu kommen, da das racemische Nitril ständig nachgeliefert wird. Bei allen anderen Nitrilen wird das enzymatisch nicht umgesetzte Nitril (= "falsches" bzw. anderes Enantiomer) vorteilhaft über eine chemische Reaktion racemisiert und dem Verfahren wieder zugeführt, um eine theoretische Ausbeute von 100 % erreichen zu können, verworfen oder aufgereinigt und chemisch unter Erhalt des Stereozentrums verseift.

Das erfindungsgemäße Verfahren wird vorteilhaft bei einer Temperatur zwischen 0°C bis 80°C, bevorzugt zwischen 10°C bis 60°C, besonders bevorzugt zwischen 15°C bis 50°C durchgeführt

Unter racemischen Nitrilen im erfindungsgemäßen Verfahren sind Nitrile zu verstehen, die aus einem 50:50 Gemisch der beiden Enantiomere oder aus einem beliebigen anderen Gemisch mit einer Anreicherung eines der beiden Enantiomere im Gemisch bestehen.

Unter chiralen Carbonsäuren sind im erfindungsgemäßen Verfahren zu verstehen, die eine Enantiomerenanreicherung zeigen. Bevorzugt werden im Verfahren Enantiomerenreinheiten von mindestens 90 %ee, bevorzugt von min. 95 %ee, besonders bevorzugt von min. 98 %ee, ganz besonders bevorzugt min. 99 %ee erreicht.

Das erfindungsgemäße Verfahren ermöglicht die Umsetzung einer großen Vielzahl von racemischen Nitrilen zu den chiralen Carbonsäuren. Im Verfahren lassen sich mindestens 25 mmol Nitril/h x mg Protein oder mindestens 25 mmol Nitril/h x g Trockengewicht der Mikroorganismen umsetzen, bevorzugt mindestens 30 mmol Nitril/h x mg Protein oder mindestens 30 mmol Nitril/h x g Trockengewicht, besonders bevorzugt mindestens 40 mmol Nitril/h x mg Protein oder mindestens 40 mmol Nitril/h x g Trockengewicht, ganz besonders bevorzugt mindestens 50 mmol Nitril/h x mg Protein oder mindestens 50 mmol Nitril/h x g Trockengewicht.

Für das erfindungsgemäße Verfahren können wachsende Zellen verwendet werden, die die erfindungsgemäßen Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Die im erfindungsgemäßen Verfahren hergestellten chiralen Carbonsäuren lassen sich vorteilhaft aus der wäßrigen Reaktionslösung über Extraktion oder Kristallisation oder über Extraktion und Kristallisation gewinnen. Hierzu wird die wäßrige Reaktionslösung mit einer Säure wie einer Mineralsäure (z.B. HCl oder H₂SO₄) oder einer organischen Säure angesäuert vorteilhaft auf pH-Werte unter 2 und anschließend mit einem organischen Lösungsmittel extrahiert. Die Extraktion kann zur Erhöhung der Ausbeute mehrfach wiederholt werden. Als organische Lösungsmittel können prinzipiell alle Lösungsmittel verwendet werden, die mit Wasser gegebenenfalls nach Zugabe von Salzen eine Phasengrenze zeigen. Vorteilhafte Lösungsmittel sind Lösungsmittel wie Toluol, Benzol, Hexan, Methyltertiärbutylether oder Essigester.

Nach Einengen der organischen Phase können die Produkte in der Regel in guten chemischen Reinheiten, das heißt größer 90 % chemische Reinheit, gewonnen werden. Nach Extraktion kann die organische Phase mit dem Produkt aber auch nur zum Teil eingeengt werden und das Produkt auskristallisiert werden. Dazu wird die Lösung vorteilhaft auf eine Temperatur von 0°C bis 10°C abgekühlt. Die Kristallisation kann auch direkt aus der organischen Lösung erfolgen. Das auskristallisierte Produkt kann nochmals in im gleichen oder in einem anderen Lösungsmittel zur erneuten Kristallisation aufgenommen werden und nochmals kristallisiert werden. Durch die anschließende mindestens einmalige Kristallisation kann die Enantiomerenreinheit des Produktes je nach Lage des Eutektikum weiter gesteigert werden.

Die chiralen Carbonsäuren können jedoch auch direkt nach Ansäuern mit einer Säure auf einen pH-Wert vorteilhaft unter 2 aus der wäßrigen Reaktionslösung auskristallisiert werden. Vorteilhaft wird dazu die wäßrige Lösung unter Erwärmen eingeengt und in ihrem Volumen um 10 bis 90 %, bevorzugt 20 bis 80 %, besonders bevorzugt 30 bis 70 % reduziert. Vorzugsweise wird die Kristallisation unter Kühlung durchgeführt. Temperaturen zwischen 0°C bis 10°C sind für die Kristallisation bevorzugt. Aus kostengründen ist die direkte Kristallisation aus der wäßrigen Lösung bevorzugt. Ebenfalls bevorzugt wird eine Aufarbeitung der chiralen Carbonsäuren über ein Extraktion und gegebenenfalls anschließender Kristallisation.

Bei diesen bevorzugten Aufarbeitungsarten läßt sich das Produkt des erfindungsgemäßen Verfahrens in Ausbeuten von 60 bis 100 %, bevorzugt von 80 bis 100 %, besonders bevorzugt von 90 bis 100 % bezogen auf das für die Reaktion eingesetzte Nitril isolieren. Das isoliert Produkt zeichnet sich durch eine hohe chemische Reinheit von > 90 %, bevorzugt > 95 % besonders bevorzugt von > 98 % aus. Weiterhin haben die Produkt eine hohe Enantiomerenreinheit, die durch die Kristallisation weiter gesteigert werden kann.

Die so gewonnenen Produkte eignen sich als Ausgangsmaterial für organischen Synthesen zur Herstellung von Pharmaka oder Agrochemikalien oder zur Racematspaltung.

Ein weiterer Gegenstand der Erfindung ist eine isolierte Nukleinsäuresequenz, die für ein Polypeptid mit Nitrilaseaktivität codiert, ausgewählt aus der Gruppe:
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 1 dargestellten Nukleinsäuresequenz ableiten,
c) Homologe der in SEQ ID NO: 1 dargestellten Nukleinsäuresequenz, die für Polypeptide Kodieren die mindestens 98% Homologie über den gesamten Sequenzbereich zu seq ID NO: 2 aufweisen ohne daß die enzymatische Wirkung der Polypeptide reduziert ist.

Unter Homologe der erfindungsgemäßen Nukleinsäuresequenz mit der Sequenz SEQ ID NO: 1 sind beispielsweise Allelvarianten zu verstehen, die mindestens 98 % Homologie über den gesamten Sequenzbereich aufweisen. Über Teilbereiche der Sequenzen können die Homologien vorteilhaft höher liegen. Die von SEQ ID NO: 1 abgeleitete Aminosäuresequenz ist SEQ ID NO: 2 zu entnehmen. Allelvarianten umfassen insbesondere funktionelle Varianten, die durch Deletion, Insertion oder Substitution von Nukleotiden aus der in SEQ ID NO: 1 dargestellten Sequenz erhältlich sind, wobei die enzymatische Aktivität der abgeleiteten synthetisierten Proteine für das Einbringen eines oder mehrerer Gene in einen Organismus jedoch erhalten nicht wesentlich reduziert sein sollte. Die Erfindung betrifft damit auch Aminosäuresequenzen, die durch die oben dargestellte Gruppe von Nukleinsäureseauenzen kodiert werden. Vorteilhaft betrifft die Erfindung Aminosäuresequenzen, die durch die Sequenz SEQ ID NO: 1 kodiert werden.

Weiterhin sind unter Homologe der SEQ ID NO: 1 beispielsweise pilzliche oder bakterielle Homologe, verkürzte Sequenzen, Einzelstrang-DNA oder RNA der codierenden und nichtcodierenden DNA-Sequenz zu verstehen. Homologe der SEQ ID NO: 1 besitzen auf DNA-Ebene eine Homologie von mindestens 60 %, bevorzugt von mindestens 70 %, besonders bevorzugt von mindestens 80 %, ganz besonders bevorzugt von mindestens 90 % über den gesamten in SEQ ID NO: 1 angegebenen DNA-Bereich.

Außerdem sind unter Homologe der SEQ ID NO: 1 Derivate wie beispielsweise Promotorvarianten zu verstehen. Die Promotoren, die den angegebenen Nukleotidsequenzen vorgeschalten sind, können durch ein oder mehrere Nukleotidaustausche, durch Insertion(en) und/oder Deletion(en) verändert sein, ohne daß aber die Funktionalität bzw. Wirksamkeit der Promotoren beeinträchtigt sind. Des weiteren können die Promotoren durch Veränderung ihrer Sequenz in ihrer Wirksamkeit erhöht oder komplett durch wirksamere Promotoren auch artfremder Organismen ausgetauscht werden.

Unter Derivaten sind auch Varianten zu verstehen, deren Nukleotidsequenz im Bereich von -1 bis -200 vor dem Startkodon oder 0 bis 1000 Basenpaare nach dem Stopkodon so verändert wurden, daß die Genexpression und/oder die Proteinexpression verändert, bevorzugt erhöht wird.

Vorteilhaft läßt sich die SEQ ID NO: 1 oder seine Homologen aus Bakterien, bevorzugt aus gram-negativen Bakterien, besonders bevorzugt aus Bakterien der Gattung Alcaligenes, ganz besonders bevorzugt aus Bakterien der Gattung und Art Alcaligenes faecalis über dem Fachmann bekannte Methoden isolieren.

SEQ ID No: 1 oder seine Homologen oder Teile dieser Sequenzen lassen sich beispielsweise mit üblichen Hybridisierungsverfahren oder der PCR-Technik aus anderen Pilzen oder Bakterien isolieren. Diese DNA-Sequenzen hybridisieren unter Standardbedingungen mit den erfindungsgemäßen Sequenzen. Zur Hybridisierung werden vorteilhaft kurze Oligonukleotide der konservierten Bereiche beispielsweise aus dem aktiven Zentrum, die über Vergleiche mit anderen Nitrilasen oder Nitrilhydratasen in dem Fachmann bekannter Weise ermittelt werden können, verwendet. Es können aber auch längere Fragmente der erfindungsgemäßen Nukleinsäuren oder die vollständigen Sequenzen für die Hybridisierung verwendet werden. Je nach der verwendeten Nukleinsäure Oligonukleotid, längeres Fragment oder vollständige Sequenz oder je nachdem welche Nukleinsäureart DNA oder RNA für die Hybridisierung verwendet werden, variieren diese Standardbedingungen. So liegen beispielsweise die Schmelztemperaturen für DNA:DNA-Hybride ca 10°C niedriger als die von DNA:RNA-Hybriden gleicher Länge.

Unter Standardbedingungen sind beispielsweise je nach Nukleinsäure Temperaturen zwischen 42 und 58°C in einer wäßrigen Pufferlösung mit einer Konzentration zwischen 0,1 bis 5 x SSC (1 X SSC = 0,15 M NaCl, 15 mM Natriumcitrat, pH 7,2) oder zusätzlich in Gegenwart von 50 % Formamid wie beispielsweise 42°C in 5 x SSC, 50 % Formamid zu verstehen. Vorteilhafterweise liegen die Hybridisierungsbedingungen für DNA:DNA-Hybride bei 0,1 x SSC und Temperaturen zwischen etwa 20°C bis 45°C, bevorzugt zwischen etwa 30°C bis 45°C. Für DNA:RNA-Hybride liegen die Hybridisierungsbedingungen vorteilhaft bei 0,1 x SSC und Temperaturen zwischen etwa 30°C bis 55°C, bevorzugt zwischen etwa 45°C bis 55°C. Diese angegebenen Temperaturen für die Hybridisierung sind beispielhaft kalkulierte Schmelztemperaturwerte für eine Nukleinsäure mit einer Länge von ca. 100 Nukleotiden und einem G + C-Gehalt von 50 % in Abwesenheit von Formamid. Die experimentellen Bedingungen für die DNA-Hybridisierung sind in einschlägigen Lehrbüchern der Genetik wie beispielsweise Sambrook et al., "Molecular Cloning", Cold Spring Harbor Laboratory, 1989, beschrieben und lassen sich nach dem Fachmann bekannten Formeln beispielsweise abhängig von der Länge der Nukleinsäuren, der Art der Hybride oder dem G + C-Gehalt berechnen. Weitere Informationen zur Hybridisierung kann der Fachmann folgenden Lehrbüchern entnehmen: Ausubel et al. (eds), 1985, Current Protocols in Molecular Biology, John Wiley & Sons, New York; Hames and Higgins (eds), 1985, Nucleic Acids Hybridization: A Practical Approach, IRL Press at Oxford University Press, Oxford; Brown (ed), 1991, Essential Molecular Biology: A Practical Approach, IRL Press at Oxford University Press, Oxford.

Unter dem erfindungsgemäßen Nukleinsäurekonstrukt sind die Nitrilasegen Sequenz SEQ ID No. 1 und seine Homologen zu verstehen, die mit einem oder mehreren Regulationssignalen vorteilhafterweise zur Erhöhung der Genexpression funktionell verknüpft wurden. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen kann die natürliche Regulation dieser Sequenzen vor den eigentlichen Strukturgenen noch vorhanden sein und gegebenenfalls genetisch verändert worden sein, so daß die natürliche Regulation ausgeschaltet und die Expression der Gene erhöht wurde. Das Nukleinsäurekonstrukt kann aber auch einfacher aufgebaut sein, das heißt es wurden keine zusätzlichen Regulationssignale vor die Sequenz SEQ ID No. 1 oder seine Homologen inseriert und der natürliche Promotor mit seiner Regulation wurde nicht entfernt. Stattdessen wird die natürliche Regulationssequenz so mutiert, daß keine Regulation mehr erfolgt und die Genexpression gesteigert wird. Das Nukleinsäurekonstrukt kann außerdem vorteilhafterweise auch eine oder mehrere sogenannte "enhancer Sequenzen" funktionell verknüpft mit dem Promotor enthalten, die eine erhöhte Expression der Nucleinsäuresequenz ermöglichen. Auch am 3'-Ende der DNA-Sequenzen können zusätzliche vorteilhafte Sequenzen inseriert werden wie weitere regulatorische Elemente oder Terminatoren. Die erfindungsgemäßen Nukleinsäuren können in einer oder mehreren Kopien im Konstrukt enthalten sein. Im Konstrukt können noch weitere Marker wie Antibiotikaresistenzen oder Auxotrophien komplementierende Gene gegebenenfalls zur Selektion auf das Konstrukt enthalten sein.

Vorteilhafte Regulationssequenzen für das erfindungsgemäße Verfahren sind beispielsweise in Promotoren wie cos-, tac-, trp-, tet-, trp-tet-, lpp-, lac-, lpp-lac-, lacI^{q-,} T7-, T5-, T3-, gal-, trc-, ara-, SP6-, λ-P_{R}- oder im λ-P_{L}-Promotor enthalten, die vorteilhafterweise in gram-negativen Bakterien Anwendung finden. Weitere vorteilhafte Regulationssequenzen sind beispielsweise in den gram-positiven Promotoren amy und SPO2, in den Hefe- oder Pilzpromotoren ADC1, MFα, AC, P-60, CYC1, GAPDH, TEF, rp28, ADH. In diesem Zusammenhang sind auch die Promotoren der Pyruvatdecarboxylase und der Methanoloxidase aus beispielsweise Hansenula vorteilhaft. Es können auch künstliche Promotoren für die Regulation verwendet werden.

Das Nukleinsäurekonstrukt wird zur Expression in einem Wirtsorganismus vorteilhafterweise in einen Vektor wie beispielsweise einem Plasmid, einem Phagen oder sonstiger DNA inseriert, das eine optimale Expression der Gene im Wirt ermöglicht. Diese Vektoren stellen eine weitere Ausgestaltung der Erfindung dar. Geeignete Plasmide sind beispielsweise in E. coli pLG338, pACYC184, pBR322, pUC18, pUC19, pKC30, pRep4, pHS1, pHS2, pPLc236, pMBL24, pLG200, pUR290, pIN-III¹¹³-B1, λgt11 oder pBdCI, in Streptomyces pIJ101, pIJ364, pIJ702 oder pIJ361, in Bacillus pUB110, pC194 oder pBD214, in Corynebacterium pSA77 oder pAJ667, in Pilzen pALS1, pIL2 oder pBB116, in Hefen 2∝M, pAG-1, YEp6, YEp13 oder pEMBLYe23 oder in Pflanzen pLGV23, pGHlac⁺, pBIN19, pAK2004 oder pDH51. Die genannten Plasmide stellen eine kleine Auswahl der möglichen Plasmide dar. Weitere Plasmide sind dem Fachmann wohl bekannt und können beispielsweise aus dem Buch Cloning Vectors (Eds. Pouwels P. H. et al. Elsevier, Amsterdam-New York-Oxford, 1985, ISBN 0 444 904018) entnommen werden.

Vorteilhafterweise enthält das Nukleinsäurekonstrukt zur Expression der weiteren enthaltenen Gene zusätzlich noch 3' und/oder 5' Terminale regulatorische Sequenzen zur Steigerung der Expression, die je nach ausgewähltem Wirtorganismus und Gen oder Gene für eine optimale Expression ausgewählt werden.

Diese regulatorischen Sequenzen sollen die gezielte Expression der Gene und der Proteinexpression ermöglichen. Dies kann beispielsweise je nach Wirtsorganismus bedeuten, daß das Gen erst nach Induktion exprimiert oder überexprimiert wird, oder daß es sofort exprimiert und/oder überexprimiert wird.

Die regulatorischen Sequenzen bzw. Faktoren können dabei vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

In einer weiteren Ausgestaltungsform des Vektors kann der das erfindungsgemäße Nukleinsäurekonstrukt oder die erfindungsgemäße Nukleinsäure enthaltende Vektor auch vorteilhafterweise in Form einer linearen DNA in die Mikroorganismen eingeführt werden und über heterologe oder homologe Rekombination in das Genom des Wirtsorganismus integriert werden. Diese lineare DNA kann aus einem linearisierten Vektor wie einem Plasmid oder nur aus dem Nukleinsäurekonstrukt oder der Nukleinsäure bestehen.

Für eine optimale Expression heterologer Gene in Organismen ist es vorteilhaft die Nukleinsäuresequenzen entsprechend des im Organismus verwendeten spezifischen "codon usage" zu verändern. Der "codon usage" läßt sich anhand von Computerauswertungen anderer, bekannter Gene des betreffenden Organismus leicht ermitteln.

Als Wirtsorganismen für die erfindungsgemäße Nukleinsäure oder dem Nukleinsäurekonstrukt kommen prinzipiell alle prokaryontischen oder eukaryontischen Organismen in Frage. Vorteilhafterweise werden als Wirtsorganismen Mikroorganismen wie Bakterien, Pilze oder Hefen verwendet. Vorteilhaft werden grampositive oder gram-negative Bakterien, bevorzugt Bakterien der Familie Enterobacteriaceae oder Nocardiaceae, besonders bevorzugt Bakterien der Gattungen Escherichia, Pseudomonas oder Rhodococcus verwendet. Ganz besonders bevorzugt ist die Gattung und Art Escherichia coli.

Der Wirtsorganismus gemäß der Erfindung enthält dabei vorzugsweise mindestens ein proteinisches Agenz zur Faltung der von ihm synthetisierten Polypeptide und insbesondere der in dieser Erfindung beschriebenen Nukleinsäuresequenzen mit Nitrilase-aktivität und/oder die dieses Agenz codierenden Gene, wobei dieses Agenz in einer Menge vorliegt, die größer ist als die, die der Grundmenge des betrachteten Mikroorganismus entspricht. Die für dieses Agenz codierenden Gene sind im Chromosom oder in extrachromosomalen Elementen wie zum Beispiel Plasmiden enthalten.

### Beispiele

### Beispiel 1: Reinigung der Nitrilase aus Alcaligenes faecalis 1650

### 1. Herstellung der Zellen

Alcaligenes faecalis 1650 wurde bei 30°C für die Dauer von 8 Stunden in Kulturmedium A unter Schütteln kultiviert.

### Kulturmedium A:

| | |
|---|---|
| Hefextrakt | 5 g/l |
| Pepton | 3,5 g/l |
| CH₃CO₂NH₄ | 5 g/l |
| KH₂PO₄ | 5 g/l |
| MgSO₄ | 0,2 g/l |
| FeSO₄ | 0,03 g/l |
| NaCl | 1 g/l |
| Butyronitril | 1 g/l |

Mit 200 ml dieser Vorkultur wurde ein 101-Fermenter mit 81 frischem Medium A beimpft. Der pH, die Temperatur, der Luftstrom und die Rührgeschwindigkeit lagen bei 7,2; 30°C, 300 l/h und 300 upm. Nach 22 Std. wurden 81 g Naßzellmasse gewonnen. Das entspricht einem Zelltrockengewicht von 3,8 g/l und einer optischen Dichte bei 600 nm von 8.

### 2. Bestimmung der enzymatischen Aktivität gegenüber Mandelonitril

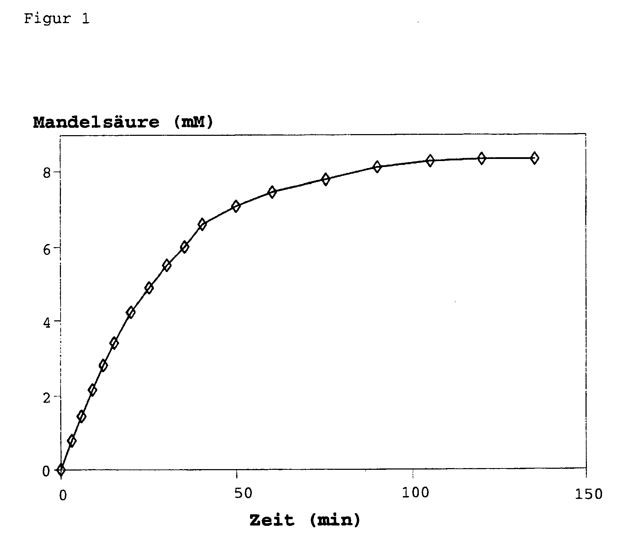

Die Zellen wurden wie in Beispiel 1 beschrieben gewonnen und in 10 mM Na/K-Phospatpuffer, pH 7,2 zweimal gewaschen. 40 mg Zelltrockengewicht wurden in 20 ml 10 mM Na/K-Phospatpuffer, pH 6,8, resuspendiert und die Reaktion durch Zugabe von 8,3 mM Mandelonitril gestartet. Die Reaktion wurde unter Schütteln bei 40°C durchgeführt. Die Kinetik der Racematspaltung wurde durch Probenentnahme und anschließender Zellabtrennung mit Hilfe der Hochleistungsflüssigkeitschromatographie (ODS Hypersil) verfolgt. Dabei wurde Mandelonitril, Benzaldehyd, Mandelsäureamid und Mandelsäure bestimmt. Die Ergebnisse sind in Figur 1 [Umsetzung von Mandelonitril (= Mandelsäurenitril) zu Mandelsäure, Batch] dargestellt. Die Bildungsgeschwindigkeit von Mandelsäure beträgt 41,3 U/g Zelltrockengewicht bei einem Umsatz von 30 %, wobei 1U definiert ist als 1 ∝mol Mandelsäure, das pro Minute bei 40°C gebildet wird.

### 3. Bestimmung der enzymatischen Selektivität gegenüber Mandelonitril

Die Zellen wurden wie in Beispiel 1 beschrieben gewonnen und in 10 mM Na/K-Phospatpuffer, pH 7,2 zweimal gewaschen. 40 mg Zelltrockengewicht wurden in 20 ml 10 mM Na/K-Phospatpuffer, pH 6,8, resuspendiert und die Reaktion durch Zugabe von 8,3 mM Mandelonitril gestartet. Die Reaktion wurde unter Schütteln bei 30°C durchgeführt. Die Kinetik wurde durch Probenentnahme und anschließende Zellabtrennung mit Hilfe der Hochleistungsflüssigkeitschromatographie (Nucleodex β-PM) verfolgt. Dabei wurde S-(+)- und R-(-)-Mandelsäure bestimmt. Die optische Reinheit der gebildeten R-(-)-Mandelsäure (ee_{R-MS}) betrug 98% bei 50 % Umsatz. Die Selektivität des Enzyms (= E) lag bei 50 % Umsatz bei 499.

### 4. Reinigung

In allen Puffern war während der Reinigung -falls nicht anders angegeben- 10 mM DTT anwesend.

### Schritt 1: Zellaufschluß

Die Zellen aus je zwei 101-Fermentationen wurden wie in Beispiel 1 beschrieben gewonnen, abzentrifugiert und zweimal mit 1 1 0,1 M Tris/HCl-Puffer, pH 7,2 gewaschen. Die Ausbeute betrug ca. 162 g Zellfeuchtmasse. Je 81 g Zellfeuchtmasse wurden in 160 ml 0,1 M Tris/HCl-Puffer, pH 7,2, resuspendiert und viermal in einem Menton-Gaulin bei 750 bar aufgeschlossen. Das Homogenat wurde dann für 30 min bei 30000 g zentrifugiert und das Pellet verworfen. Der Überstand (140 ml) hatte eine Restaktivität von 73 % wie in Tab. 1 dargestellt.

### Schritt 2: Ionenaustauschchromatographie

Der Überstand wurde mit Puffer A (20 mM Tris/HCl, pH 8,5) auf 400 ml verdünnt und nochmals bei 23000 g für 20 min zentrifugiert. 350 ml wurden dann auf eine Q-Sepharose Säule (Durchmesser 5 cm, Höhe 22 cm, Volumen 432 ml, Q-Sepharose Fast Flow von Pharmacia) in Puffer A aufgetragen. Bei einem Fluß von 20 ml/min wurde zunächst mit 10 % Puffer B (wie Puffer A mit 1 M NaCl) gewaschen (gesamtes Auftrags- und Waschvolumen entsprach 1,5 1). Im Verlauf von 90 min wurde linear das Verhältnis bis zu 60 % B gesteigert. Von 91 bis 120 min wurde dann mit 100 % Puffer B gewaschen. Es wurden 100 40ml-Fraktionen gesammelt. Die Nitrilase eluierte zwischen den Fraktionen 50 und 60. Die Fraktionen wurden vereinigt und durch Ultrafiltration über eine 10 kDa Membran (Amicon) auf ein Volumen von 10 ml aufkonzentriert.

### Schritt 3: Molekularsiebchromatographie

Das Konzentrat aus der Ionenaustauschchromatographie (Schritt 2) wurde in zwei Portionen zu je 5 ml durch Molekularsiebchromatographie (Superdex 200 prep. grade, Pharmacia, Trennbereich 10 bis 600 kDa, 2,6 cm Durchmesser, 60 cm Höhe, 325 ml Volumen) weiter gereinigt. Die Detektion erfolgte bei 280 nm. Die Säule war in 20 mM Phosphatpuffer, pH 7,4, 5 mM DTT und 150 mM NaCl äquilibriert und wurde mit einem Fluß von 1,5 ml/min betrieben. Es wurden 40 Fraktionen gesammelt. Die Nitril-verseifende Aktivität befand sich in den Fraktionen 3 bis 5.

### Schritt 4: Ionenaustauschchromatographie

Die vereinigten Fraktionen aus der Molekularsiebchromatographie (Schritt3) wurden durch Ionenaustauschchromatographie über eine Mono Q Säule (Säulenvolumen 1 ml, Mono Q HR515, Pharmacia) weiter gereinigt. Als Puffer A diente 20 mM Tris/HCl, pH 8,5, 5 mM DTT, als Puffer B der gleiche Puffer wie in A mit 1 M NaCl. Die Flußgeschwindigkeit betrug 1 ml/min. Die auf eine Leitfähigkeit von ca 6 mS/cm verdünnte Wertfraktion aus der Molekularsiebchromatographie (ca. 100 ml) wurde direkt auf die Mono Q Säule gegeben und das Protein so adsorbiert. Die Säule wurde nach dem Auftrag mit 5 % Puffer B gewaschen. Die Säule wurde in 30 min mit einem Gradienten von 5 % bis 40 % B eluiert, gefolgt von 100 % B für 10 Minuten. Die Elution der Nitrilase erfolgte in Fraktion 17 und 18 des Gradienten.

Die Schritte 1 - 4 der Reinigung werden in Tabelle I wiedergegeben.

**Tabelle I: Reinigungsschema**

| Probe | Vol. [ml] | Aktivit ät [U/l] | Gesamta ktivitä t [mU] | Ausbeut e [%] | Protein [mg/ml] | Gesamtp rotein [mg] | Spez. Aktivit ät [U/g] |
|---|---|---|---|---|---|---|---|
| vor Aufschl uβ | 160 | 480 | 76800 | 100 | - | - | - |
| nach Aufschl uβ | 140 | 400 | 56000 | 72,9 | - | - | - |
| Q-Sepharose | | | | | | | |
| Auftrag | 140 | 192 | 26880 | 35 | 12,4 | 1736 | 15 |
| WF | 400 | 77 | 30800 | 40,1 | 0,26 | 104 | 296 |
| Superdex 200 | | | | | | | |
| Auftrag | 9,5 | >378 | >3591 | 4,7 | 2,41 | 22,90 | >157 |
| WF | 43 | 59 | 2537 | 3,3 | 0,21 | 9,03 | 281 |
| MonoQ | | | | | | | |
| Auftrag | 100 | 4,8 | 480 | 0,6 | 0,06 | 6,33 | 76 |
| WF | 4 | >77 | 308 | 0,4 | 0,19 | 0,76 | >405 |

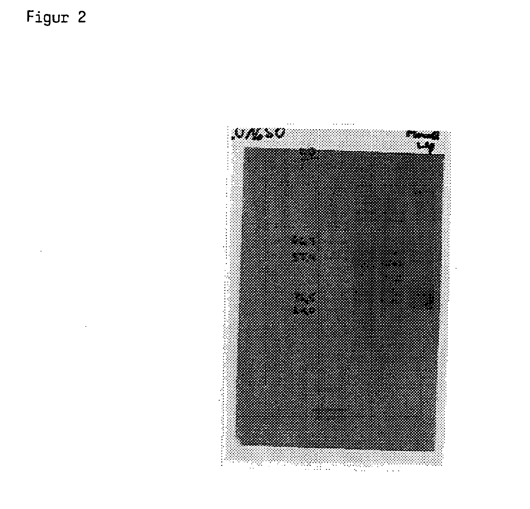

Die Wertfraktionen (= WF, Tabelle I) der Molekularsiebchromatographie (Schritt 3) und Ionenaustauschchromatographie über Mono Q (Schritt 4) sind über SDS-PAGE aufgetrennt worden wie in Figur 2 dargestellt.

### Schritt 5: Reversed-Phase (RP)-Hochflüssigkeitschomatographie

Die Wertfraktion (Fraktion 17 und 18) der Mono Q Chromatographie (Schritt 4) wurde durch RP-Chromatographie auf Homogenität überprüft und zur Vorbereitung einer Trypsinspaltung weiter gereinigt. Zur Trennung wurde eine Säule (3 cm) von Abimed an einem Hewlett-Packard Gerät (HP 1090) eingesetzt. Als Laufmittel diente Puffer A: Wasser mit 0,1 % TFA und Puffer B: Acetonitril mit 0,1 % TFA. Injektionsvolumen 0,1 ml, Flußgeschwindigkeit 0,5 ml/min. Der Elutionsgradient hatte.folgenden Verlauf:

| Minute | % Puffer A | % Puffer B |
|---|---|---|
| 0 | 80 | 20 |
| 2 | 80 | 20 |
| 22 | 30 | 70 |
| 22,1 | 0 | 100 |
| 24 | 0 | 100 |
| 25 | 100 | 0 |
| 30 | 100 | 0 |

Die Nitrilase eluierte zwischen 12 und 13 Minuten. Im SDS-PAGE entspricht das einer 37 kDa-Bande. Diese Bande wurde ansequenziert. Es wurde der Sequenzer "494 Procise Protein Sequenzer" der Firma Applied Biosystems verwendet. Die so erhaltene N-terminale Sequenz von 39 Aminosäuren wird im Folgenden mit SEQ ID NO : 3 bezeichnet. Die Sequenz ist in der beigefügten Liste der Sequenzen aufgeführt und lautet: Met Gln Thr Arg Lys Ile Val Arg Ala Ala Ala Val Gln Ala Ala Ser Pro Asn Tyr Asp Leu Ala Thr Gly Val Asp Lys Thr Ile Glu Leu Ala Arg Gln Ala Arg Asp Glu Gly.

### Herstellung tryptischer Peptide

Die Probe aus der Mono Q Chromatographie (Schritt 4) wurde wie folgt vorbehandelt: das Protein (ca 0,6 mg) wurde durch 12,5 % TCA gefällt und das Pellet dreimal mit 1 ml Ether/Ethanol (1:1) gewaschen. Das Pellet wurde in 0,2 ml 6 M Guanidin HCl, 25 mM Tris/HCl, pH 8,5 gelöst. Zu dieser Lösung wurden 2,6 ∝1 einer 1 M DTT-Lösung zur Reduktion der Disulfitbrücken gegeben. Die Probe wurde eine Stunde in Dunkelheit geschüttelt. Danach wurde das Protein mit 1,5 ∝1 einer 4-Vinylpyridinlösung (35 %) für 2 Stunden in Dunkelheit umgesetzt. Die Reaktion wurde durch Inkubation für 1 Stunde mit 2,6 ∝1 einer 1 M DTT-Lösung beendet. Das vinylpyrrilidierte Enzym wurde wie oben beschrieben durch RP-HPLC gereinigt. Die Retentionszeit betrug nun zwischen 10 und 11 Minuten. Die Wertfraktion, identifiziert durch ihr Molekulargewicht, wurde gesammelt und auf 0,02 ml aufkonzentriert. Dazu wurden 0,01 ml Acetonitril und 0,1 M Tris/HCl, pH 8,5 ad 0,2 ml gegeben. Zur Korrektur des pH-Wertes wurden noch ca. 0,05 ml 0,1 M NaOH zugesetzt. Die Probe (0,3 mg geschätzte Proteinmenge) wurde mit 0,032 ml einer 1 mg/ml Trypsinlösung in 0,1 M Tris/HCl, pH 8,5, 5 % Acetonitril, versetzt und über Nacht bei 37°C inkubiert. Der Verdau wurde mit 0,01 ml Essigsäure gestoppt und dann zentrifugiert. Der Überstand wurde durch RP-HPLC auf C18 getrennt. (Laufsystem: Puffer A: Wasser, 0,1 TFA, Puffer B: Acetonitril, 0,1 % TFA). Peptide (Detektion 205 nm und 280 nm) wurden gesammelt und sequenziert. Es wurde der Sequenzer "494 Procise Protein Sequenzer" der Firma Applied Biosystems verwendet. Die interne Peptidsequenz von 21 Aminosäuren wird im folgenden mit SEQ ID NO : 4, die interne Peptidsequenz von 11 Aminosäuren mit SEQ ID NO : 5 bezeichnet. SEQ ID NO : 4 und 5 sind in der beigefügten Liste der Sequenzen aufgeführt und lauten:
SEQ ID NO : 4
Glu Glu Ala Pro Glu Gln Gly Val Gln Ser Lys Ile Ala Ser Val Ala Ile Ser His Pro Gln
SEQ ID NO : 5
Glu Glu Ala Pro Glu Gln Gly Val Gln Ser Lys

### 6. Aktivität der gereinigten Nitrilase gegenüber Mandelonitril

Die Aktivität der gereinigten Nitrilase gegenüber Mandelonitril wurde wie in Beispiel 2 beschrieben untersucht. Die spezifische Aktivität des gereinigten Proteins gegenüber Mandelonitril lag bei 12380 U/g Protein.

### Beispiel 2: Klonierung der Nitrilase aus Alcaligenes faecalis 1650

Aus den in Beispiel 1 dargestellten Peptidsequenzen SEQ ID NO : 3 und 4 wurden Nukleotidsonden abgeleitet und synthetisiert. Von der SEQ ID NO : 3, der N-terminalen Peptidsequenz, war die abgeleitete Nukleotidsonde ein 23 mer, 64 mal degeneriert (in der Sequenz der Nukleotidsonde wird A, C, G oder T durch N ersetzt; A oder G durch R; C oder G durch S). Durch den hohen Prozentanteil an GC der in der Literatur beschriebenen Stämme Alcaligenes (Wada et al., 1992, Nucl. Acids Res., 20, 2111-2118) waren im Falle des Glutamins und des Isoleucins die Auswahl der dritten Position des Codons vorgegeben. Die Nukleotidsonde, die im Folgenden mit SEQ ID NO : 6 bezeichnet wird, stellt den 5'-Primer für die nachfolgende PCR dar, wobei S = C oder G und N = A, C, G oder T bedeutet, und lautet:
SEQ ID NO : 6
5'-ATGCAGACNAGNAARATCGTSCG-3'

Von SEQ ID NO : 4, der internen Peptidsequenz, wurde ein 20 mer als Nukleotidsonde abgeleitet, 256 mal degeneriert (in der Sequenz der Nukleotidbasen wird A, C, G oder T durch N ersetzt; A oder G durch R; C oder G durch S). Durch den hohen Prozentanteil an GC der Stämme Alcaligenes war im Falle des Lysins die Auswahl der dritten Position des Codons vorgegeben. Diese Nukleotidsonde stellt den 3'-Primer für die nachfolgende PCR dar und wird im Folgenden mit SEQ ID NO : 7 bezeichnet. Sie ist in der beigefügten Liste der Sequenzen aufgeführt und lautet:
SEQ ID NO : 7
5'-TNGCSACNGANGCRATCTTG-3'

Mit Hilfe dieses Primerpaars, SEQ ID NO : 6 und 7, wurde die PCR an chromosomaler DNA aus Alcaligenes faecalis 1650 durchgeführt. Die Isolierung chromosomaler DNA erfolgte nach Zelllyse mit Lysozym und Proteinase K-Behandlung nach der dem Fachmann bekannten klassischen Methode (Ausubel, F. M. et al. (1994) Current protocols in molecular biology, John Wiley and Sons).

Unter Verwendung der Pwo-Polymerase beinhaltete die PCR eine Denaturierung für 3 min bei 95°C; 35 Zyklen mit einer Denaturierung für 1 min bei 95°C, einer Primeranlagerung für 1 min 30 sec bei 58°C und eine Polymerisation für 1 min 30 sec bei 72°C; und einer Abschlußpolymerisation für 5 min bei 72°C.

Unter diesen Bedingungen wurde aus der chromosomalen DNA aus Alcaligenes faecalis 1650 ein etwa 1 kb großes Fragment amplifiziert. Zur Klonierung des PCR-Produktes wurde an die bereits erwähnten Primer je eine XbaI-Restriktions-schnittstelle und zwei zusätzliche Nukleotide angehängt (5'-AATCTAGA bzw. 5'-ATTCTAGA) und die PCR-Reaktion unter den oben genannten Bedingungen wiederholt. Erneut wurde ein etwa 1 kb-großes Fragment amplifiziert, das nach Reinigung und XbaI-Verdau in analog verdauten pUC18 ligiert wurde. Nach Transformation von E. coli JM109 und Isolierung des resultierenden Plasmids wurde die DNA durch Sequenzierung und anschließenden genomischen Southern Blot verifiziert. Die molekularbiologischen und mikrobiologischen Methoden zur Isolierung des kompletten Nitrilase-Gens (nit) erfolgte nach den dem Fachmann bekannten klassischen Methoden. Die komplette Nitrilase-Sequenz ist in SEQ ID NO: 1 dargestellt.

### Beispiel 3: Homologie mit anderen Proteinen, Identifizierung der homologen Sequenz

Der Vergleich mit Sequenzen aus der Proteindatenbank SWISSPROT zeigte, daß das Nitrilasegen in dieser Erfindung 11 bis 96 % Homologie zu bekannten Nitrilasen auf Aminosäureebene besitzt. Die höchste Sequenzhomologie wurde zu der Arylacetonitrilspezifischen Nitrilase aus Alcalignes faecalis JM3 (Nagasawa et al., Eur. J. Biochem. 1990, 194, 765-772) gefunden. Die beiden Nitrilasegene weisen eine Identität von 93,2 % auf Nukleotidebene über einen Bereich von 1071 bp auf. Die abgeleitete Aminosäuresequenz weist eine Identität von 96,1 % über einen Bereich von 356 Aminosäuren auf. Die geringste Homologie von 11,4 % über einen Bereich von 534 Aminosäuren wurde zu der Nitrilase aus Rhodococcus erythropolis SK92 (EP-A-0 719 862) gefunden.

### Beispiel 4: Heterologe Expression der Nitrilase in E. coli

Zur Klonierung in den Expressionsvektor pJOE2702 wurde das nit-Gen amplifiziert. Dabei wurde als 5'-Primer für die PCR die o.g. SEQ ID NO : 3 ausgewählt, wobei an das 5'-nit-Ende eine mit dem Translationsstart überlappende NdeI-Schnittstelle angefügt wurde. Dieser Primer wird im Folgenden als SEQ ID NO : 8 bezeichnet und ist in der beigefügten Liste der Sequenzen aufgeführt. Als 3'-Primer wurde ein 24 mer aus dem 3'-Bereich des nit-Gens ausgewählt, bei dem eine an das Stopcodon angrenzende BamHI-Schnittstellen angefügt wurde. Er wird im Folgenden als SEQ ID NO : 9 bezeichnet und ist in der nachfolgenden Liste der Sequenzen aufgeführt.
5'-TTAATCATATGCAGACAAGAAAAATCGTCCG-3' (= SEQ ID NO: 8) 5'-AAGGATCCTCAAGACGGCTCTTGCACTAGCAG-3' (= SEQ ID NO : 9)

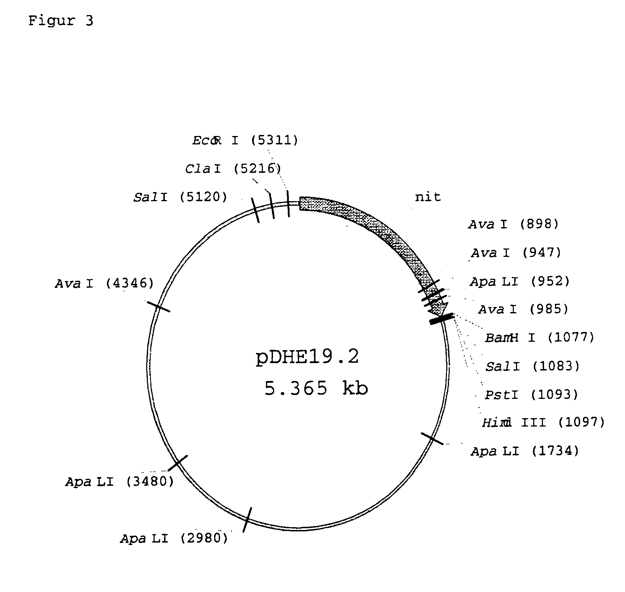

Unter Verwendung der Pwo-Polymerase beinhaltete die PCR eine Denaturierung für 3 min bei 94°C; 25 Zyklen mit einer Denaturierung für 1 min bei 93°C, einer Primeranlagerung für 1 min 30 sec bei 55°C und einer Polymerisation für 1 min 30 sec bei 72°C bzw. einer Abschlußpolymerisation für 5 min bei 72°C. Das erhaltene PCR-Fragment wurde gereinigt, mit NdeI/BamHI verdaut und in analog verdauten Vektor pJOE2702 (Volff et al., 1996, Mol. Microbiol., 21(5), 1037-1047) integriert. Das resultierende Plasmid wurde mit pDHE 19.2 bezeichnet und ist in Figur 3 dargestellt. Durch die Integration über die NdeI/BamHI-Schnittstellen steht das nit-Gen in dem Plasmid pDHE19.2 unter Transkriptionskontrolle des in pJOE2702 enthaltenen Promotors *rhaₚ,* der aus dem positiv regulierten L-Rhamnose-Operon rhaBAD in E. coli (Egan & Schleif, 1994, J.Mol. Biol., 243, 821-829) stammt. Die Transkriptionstermination des nit-Gens und die Translationsinitiation erfolgen ebenfalls über Vektorsequenzen. Daneben enthält das Plasmid noch ein Gen, das die Ampicillin-Resistenz *Ap^{R}* verleiht.

Die heterologe Expression der Nitrilase wurde bei dem das Plasmid pDHE19.2 enthaltenden Stamm E. coli JM109 gezeigt. Zu diesem Zweck wurde der Stamm JM109 (pDHE19.2) im Kulturmedium TB bei 37°C mit 100 ∝g/ml Ampicillin (Tartof, Hobbs 1987) unter Schütteln angezogen. Bei einer OD₆₀₀ von 1,7 wurde die Kultur 1:200 in frisches TB-Medium, das zur Induktion der Nitrilase 0,2% (w/v) L-Rhamnose enthielt, überimpft und bei 30°C unter Schütteln kultiviert. Nach 8 Stunden wurden die Zellen geerntet, mit 10 mM Na/K-Phosphatpuffer, pH 7,2, gewaschen, in demselben Puffer entsprechend einer OD₆₀₀ von 10 resuspendiert und nach Ultraschallbehandlung aufgeschlossen.

### Beispiel 5: Bestimmung der Nitrilase-Aktivität des rekombinaten Stamms E. coli JM109 (pDHE19.2)

### 1. Herstellung der Zellen

E. coli JM109 (pDHE19.2) wurde bei 37°C für die Dauer von 6 Stunden in TB-Medium + 100 ∝g/ml Ampicillin unter Schütteln kultiviert. Bei einer OD₆₀₀ von 4 wurde mit 100 ml dieser Vorkultur ein 101-Fermenter mit 81 frischem TB-Medium + 100 µg/ml Ampicillin + 2 g/l L-Rhamnose beimpft. Der pH, die Temperatur, der Luftstrom und die Rührgeschwindigkeit lagen bei 7,2, 30°C, 300 l/h und 400-650 upm. Nach 16 Stunden wurden die Zellen geerntet. Zu diesem Zeitpunkt betrug die optische Dichte bei 600 nm 18, was einem Zelltrockengewicht von 7,8 g/l entsprach.

### 2. Bestimmung der spezifischen Aktivität gegenüber Mandelonitril

Die Zellen wurden wie in Beispiel 1 beschrieben gewonnen und in 10 mM Na/K-Phospatpuffer, pH 7,2 gewaschen. 2 mg Zelltrocken gewicht wurden in 1 ml 10 mM Na/K-Phospatpuffer, pH 7,2, resuspendiert und die Reaktion durch Zugabe von 8,3 mM Mandelonitril gestartet. Die Reaktion wurde unter Schütteln bei 40°C durchgeführt. Die Kinetik wurde über Probenentnahme und anschließende Hochleistungsflüssigkeitschromatographie (ODS Hypersil) verfolgt. Dabei wurde Mandelonitril, Benzaldehyd, Mandelsäureamid und Mandelsäure bestimmt. Die Bildungsgeschwindigkeit von Mandelsäure beträgt 403 U/g Zelltrockengewicht bei einem Umsatz von 30 %, wobei 1U definiert ist als 1 ∝mol Mandelsäure, das pro Minute bei 40°C gebildet wird.

### Beispiel 6: Synthese von R-Mandelsäure über Verseifung von Mandelonitril mit Hilfe von E. coli JM109 (pDHE19.2) in Suspension

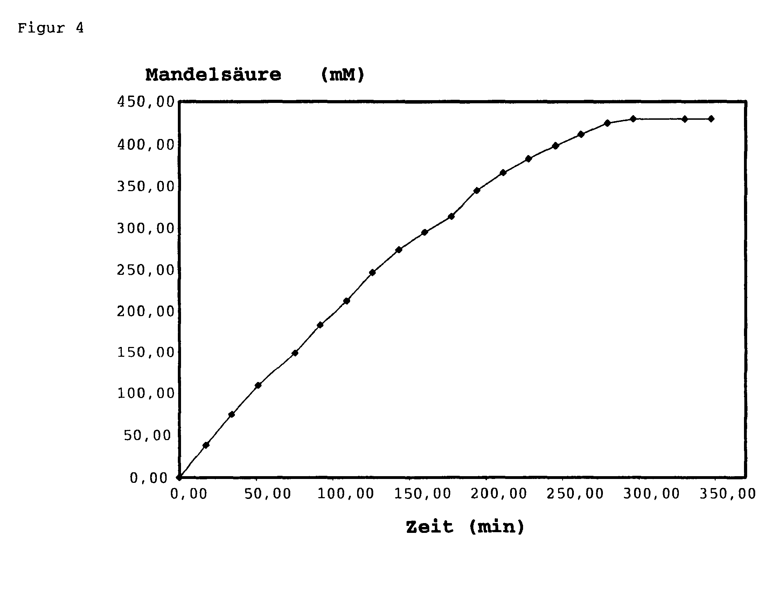

In einem Volumen von 11 10 mM Na/K-Phosphatpuffer, pH 7,2, der den Stamm E. coli JM109 (pDHE19.2) in einer Konzentration von 2 g/l enthielt, wurde bei 40°C unter Rühren mit einem Blattrührer über 10 Stunden Mandelonitril in einer Konzentration von 1,3 g/l zudosiert. Die Dosierung wurde über den Nitril-Verbrauch reguliert. Die Bildungsgeschwindigkeit von R-Mandelsäure wurde wie in Beispiel 5 beschrieben verfolgt. Die Ergebnisse sind in Figur 4 dargestellt.

### Beispiel 7: Gewinnung von R-Mandelsäure über Extraktion aus dem Reaktionsansatz der Mandelonitril-Verseifung durch E. Coli JM109 (pDHE19.2) in Suspension

Der in Beispiel 6 erhaltene, wässrige Reaktionsansatz an Mandelsäure wurde von den Zellen über Zentrifugation befreit, mit einer Säure auf pH 2 gestellt und dreimal mit Methyltertiärbutylether (MTBE) extrahiert. Nach Abdampfen des organischen Lösungsmittels des Mandelsäureextraktes wurden die so erhaltenen, weißen Mandelsäurekristalle rückgelöst und auf chemische und optische Reinheit über Hochleistungsflüssigkeitschromatographie untersucht. Die chemische Reinheit lag bei 99 %, die optische Reinheit der R-Mandelsäure bei 97,4 % ee.

### Beispiel 8: Gewinnung von R-Mandelsäure über Kühlungs-Kristallisation aus dem Reaktionsansatz der Mandelonitril-Verseifung durch E. coli JM109 (pDHE19.2) in Suspension

Der in Beispiel 6 erhaltene, wässrige Reaktionsansatz an Mandelsäure wurde von den Zellen über Zentrifugation befreit, unter Erwärmung und Rührung auf 40 % des Ausgangsvolumens aufkonzentriert und mit einer Säure auf pH 2 gestellt. Durch Abkühlung im Eisbad wurde die Mandelsäure auskristallisiert und die so erhaltenen, weißen Mandelsäurekristalle über eine Nutsche abgesaugt und getrocknet. Die Kristalle wurden rückgelöst und über Hochleistungsflüssigkeitschromatographie auf chemische und optische Reinheit untersucht. Die chemische Reinheit lag bei 99,1 %, die optische Reinheit der R-Mandelsäure lag bei 99,8 % ee.

### Beispiel 9: Umsetzung verschiedener Nitrile

Mit dem E. coli Stamm (siehe Beispiel 6) oder mit dem Ausgangsstamm Alcaligenes wurden verschieden Nitrile umgesetzt. Die Alcaligenes-Zellen wurden in 400 ml Alcaligenes-Medium (siehe oben Medium A) bei 30°C und 160 Upm für 16 Stunden (= h) angezogen. Die Zellernte erfolgte durch Zentrifugation (30 min, 4°C und 5000 Upm). Je 150 ∝1 einer Zellsuspension wurden pro Well in eine Mikrotiterplatte pipetiert. Die Platte wurde anschließend zentrifugiert. Der Überstand wurde abgesaugt und die Zellpellets zweimal mit Na₂HPO₄ (1,42 g/l in Finnaqua, pH 7,2) gewaschen. Anschließend wurde die Substratlösung (150 ∝1) zupipettiert und die Zellen erneut resuspendiert. Je eine 12-er Reihe der Mikrotiterplatte wurde mit einem Substrat versetzt. Als Kontrolle wurde eine Reihe mit der Substratlösung ohne Zellen genommen (= Leerwert).

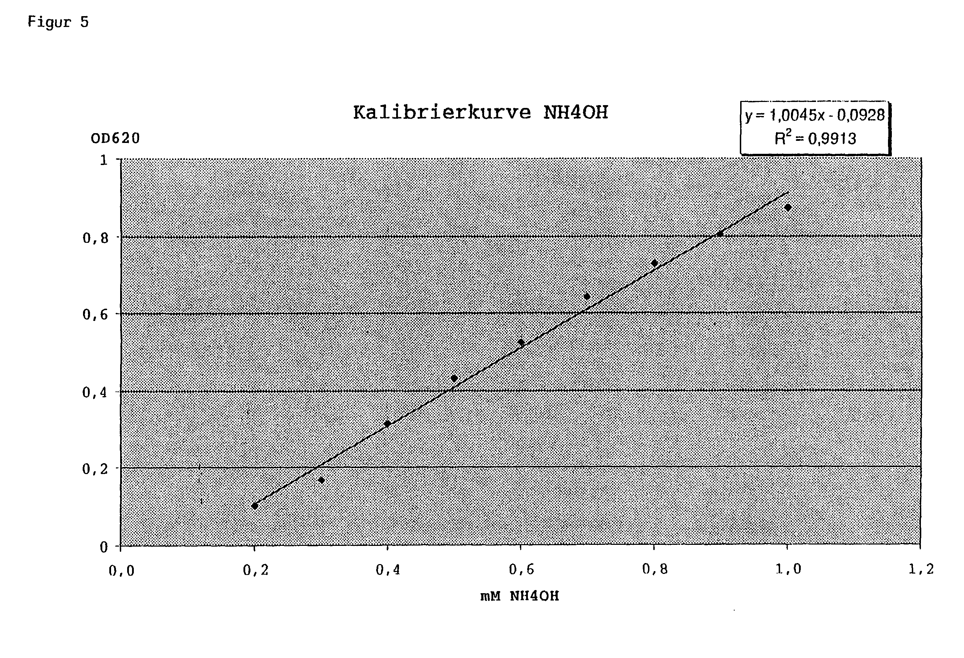

Die Mikrotiterplatten wurden bei 30°C und 200 Upm für 2 Stunden im Schüttelinkubator belassen. Danach wurden die Zellen abzentrifugiert und im Überstand die entstandene Menge an NH₄-Ionen mit Hilfe der Biomek-Geräts bestimmt. Die Messung erfolgte bei 620 nm gegen eine Eichkurve, die mit verschiedenen NH₄OH-Lösungen erstellt wurden war (siehe Figur 5). Als Substrate wurden Mandelonitril (= 1), 2-Phenylpropionitril (= 2), 2-Phenylbutyronitril (= 3), Benzylcyanid (= 4), 4-Chlorbenzylcyanid (= 5), 4-Brombenzylcyanid (= 6), Propionitril (= 7), 2-Methylbutyronitril (= 8, 2-Cyanobutan), Geranonitril (= 9), Valeronitril (= 10), 3-Cyanpyridin (= 11), 3-Biphenyl-2-hydroxy-butyronitril (= 12), 4-Flourbenzylcyanid (= 13, 4-Fluorophenylacetronitril) und α-(3-Heptyl)-nitro-triacetonitril (= 14) verwendet. Die Substrate wurden 0,2 molar in Methanol angesetzt und von dieser Stammlösung ausgehend mit Na₂HPO₄ (1,42 g/l in Finnaqua, pH 7,2) auf 10 mM verdünnt. Die Zellsuspensionen wurden auf 2 g/l Biotrockenmasse standardisiert. Tabelle II gibt die Mittelwerte einer Mikrotiterplattenreihe bei der Umsetzung wieder.

**Tabelle II: Umsetzung verschiedener Nitrile mit Nitrilase 1650**

| Substrat-Nr. | ∝mol/l | Aktivität | % Umsatz |
|---|---|---|---|
| 1 | 2141,2 | 8,9 | 86,3 |
| 2 | 1001,1 | 4,1 | 70,2 |
| 3 | 24,4 | 0,1 | 44,3 |
| 4 | 2210,5 | 9,2 | 100 |
| 5 | 2136,3 | 8,9 | 100 |
| 6 | 1500,8 | 6,2 | 100 |
| 7 | 4,9 | 0,02 | NA |
| 8 | - | - | NA |
| 9 | - | - | NA |
| 10 | 113,4 | 0,47 | NA |
| 11 | - | - | NA |
| 12 | - | - | NA |
| 13 | 2222,9 | 9,2 | 100 |
| 14 | 84,8 | 0,35 | 44,1 |

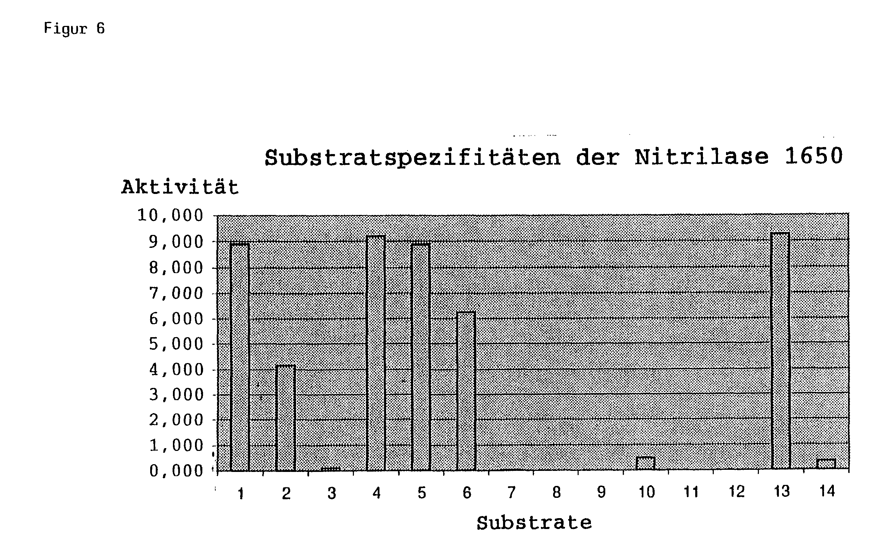

Figur 6 gibt die Ergebnisse der Umsetzung als Aktivitätswerte wieder.

## Patentansprüche

1. Isolierte Nukleinsäuresequenz, die für ein Polypeptid mit Nitrilaseaktivität codiert, ausgewählt aus der Gruppe:
a) einer Nukleinsäuresequenz mit der in SEQ ID NO: 1 dargestellten Sequenz,
b) Nukleinsäuresequenzen, die sich als Ergebnis des degenerierten genetischen Codes von der in SEQ ID NO: 1 dargestellten Nukleinsäuresequenz ableiten,
c) Homologe der in SEQ ID NO: 1 dargestellten Nukleinsäuresequenz, die für Polypeptide Kodieren die mindestens 98% Homologie über den gesamten Sequenzbereich zu SEQ ID NO : 2 aufweisen ohne daß die enzymatische Wirkung der Polypeptide reduziert ist.

2. Aminosäuresequenz codiert durch eine Nukleinsäuresequenz gemäß Anspruch 1.

3. Aminosäuresequenz nach Anspruch 2, codiert durch die in SEQ ID NO: 1 dargestellte Sequenz.

4. Nukleinsäurekonstrukt enthaltend eine Nukleinsäuresequenz gemäß Anspruch 1, wobei die Nukleinsäuresequenz mit einem oder mehreren Regulationssignalen verknüpft ist.

5. Vector enthaltend eine Nukleinsäuresequenz gemäß Anspruch 1 oder ein Nukleinsäurekonstrukt gemäß Anspruch 4.

6. Mikroorganismus enthaltend mindestens eine eingebrachte Nukleinsäuresequenz gemäß Anspruch 1 oder mindestens ein eingebrachtes Nukleinsäurekonstrukt gemäß Anspruch 4.

7. Mikroorganismus nach Anspruch 6, wobei es sich bei dem Mikroorganismus um ein Bakterium der Gattungen Escherichia, Pseudomonas oder Alcaligenes handelt.

8. Verfahren zur Herstellung von chiralen Carbonsäuren der allgemeinen Formel I 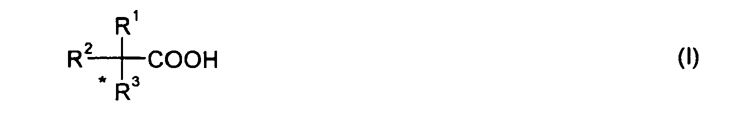 **dadurch gekennzeichnet, daß** man racemische Nitrile der allgemeinen Formel II 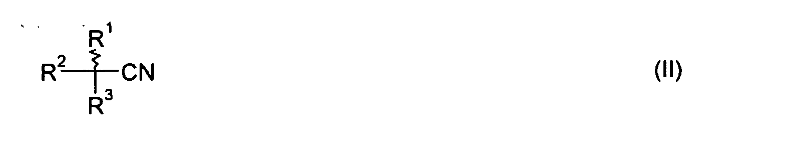 in Gegenwart einer Aminosäuresequenz gemäß Anspruch 2 oder 3 oder einem wachsenden, ruhenden oder aufgeschlossenen Mikroorganismus gemäß Anspruch 6 oder 7 umsetzt und wobei mindestens 25 mmol Nitril/h pro mg Protein oder 25 mmol Nitril/h pro g Trockengewicht zu den chiralen Carbonsäuren umgesetzt werden,
wobei die Substituenten und Variablen in den Formeln I und II folgende Bedeutung haben:
* ein optisch aktives Zentrum
R¹,R², R³ unabhängig voneinander Wasserstoff, substituiertes oder unsubstituiertes, verzweigtes oder unverzweigtes C₁-C₁₀-Alkyl-, C₂-C₁₀-Alkenyl-, substituiertes oder unsubstituiertes Aryl-, Hetaryl-, OR⁴ oder NR⁴R⁵ und wobei die Reste R¹, R² und R³ immer unterschiedlich sind,
R⁴ Wasserstoff, substituiertes oder unsubstituiertes, verzweigtes oder unverzweigtes C₁-C₁₀-Alkyl-, C₂-C₁₀-Alkenyl-, C₁-C₁₀-Alkylcarbonyl-, C₂-C₁₀-Alkenylcarbonyl-, Aryl-, Arylcarbonyl-, Hetaryl- oder Hetarylcarbonyl-,
R⁵ Wasserstoff, substituiertes oder unsubstituiertes, verzweigtes oder unverzweigtes C₁-C₁₀-Alkyl-, C₂-C₁₀-Alkenyl-, Aryl- oder Hetaryl-.

9. Verfahren nach Anspruch 8, **dadurch gekennzeichnet, daß** einer der Substituenten R¹, R² oder R³ OR⁴ bedeutet.

10. Verfahren nach Anspruch 8 oder 9, **dadurch gekennzeichnet, daß** einer der Substituenten R¹, R² oder R³ Aryl- bedeutet.

11. Verfahren nach den Ansprüchen 8 bis 10, **dadurch gekennzeichnet, daß** das Verfahren in wäßriger Reaktionslösung bei einem pH zwischen 4 bis 11 durchgeführt wird.

12. Verfahren nach den Ansprüchen 8 bis 11, **dadurch gekennzeichnet, daß** im Verfahren 0,01 bis 10 Gew.-% Nitril oder 0,01 bis 10 Gew.-% eines entsprechenden Aldehyds oder Ketons und 0,01 bis 10 Gew.-% Blausäure umgesetzt werden.

13. Verfahren nach den Ansprüchen 8 bis 12, **dadurch gekennzeichnet, daß** das Verfahren bei einer Temperatur zwischen 0°C bis 80°C durchgeführt wird.

14. Verfahren nach den Ansprüchen 8 bis 13, **dadurch gekennzeichnet, daß** die chirale Carbonsäure über Extraktion oder Kristallisation oder Extraktion und Kristallisation in Ausbeuten von 60 bis 100 % aus der Reaktionslösung gewonnen wird.

15. Verfahren nach den Ansprüchen 8 bis 14, **dadurch gekennzeichnet, daß** die chirale Carbonsäure eine optische Reinheit von mindestens 90 %ee besitzt.

## Claims

1. An isolated nucleic acid sequence which codes for a polypeptide having nitrilase activity, selected from the group of:
a) a nucleic acid sequence having the sequence depicted in SEQ ID NO: 1,
b) nucleic acid sequences which are derived from the nucleic acid sequence depicted in SEQ ID NO: 1 as a result of the degeneracy of the genetic code,
c) homologs of the nucleic acid sequence depicted in SEQ ID NO: 1, which code for polypeptides having the at least 98% homology, over the entire sequence range, to SEQ ID NO: 2, with no reduction in the enzymatic action of the polypeptides.

2. An amino acid sequence encoded by a nucleic acid sequence according to claim 1.

3. The amino acid sequence according to claim 2, encoded by the sequence depicted in SEQ ID NO: 1.

4. A nucleic acid construct comprising a nucleic acid sequence according to claim 1, the nucleic acid sequence being linked to one or more regulatory signals.

5. A vector comprising a nucleic acid sequence according to claim 1 or a nucleic acid construct according to claim 4.

6. A microorganism comprising at least one introduced nucleic acid sequence according to claim 1 or at least one introduced nucleic acid construct according to claim 4.

7. The microorganism according to claim 6, where the microorganism is a bacterium of the genera Escherichia, Pseudomonas or Alcaligenes.

8. A process for preparing chiral carboxylic acids of the general formula I 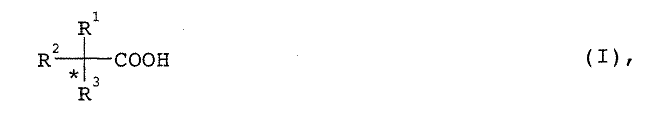 which comprises converting racemic nitriles of the general formula II 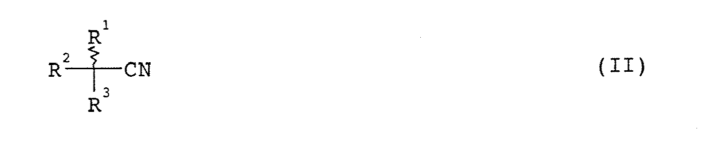 in the presence of an amino acid sequence according to claim 2 or 3 or a growing, dormant or disrupted microorganism according to claim 6 or 7, and where at least 25 mmol of nitrile are converted per h and per mg of protein, or 25 mmol of nitrile are converted per h and per g of dry weight, into the chiral carboxylic acids,
where the substituents and variables in the formulae I and II have the following meanings:
* an optically active center
R¹,R², R³ independently of one another hydrogen, substituted or unsubstituted, branched or unbranched C₁-C₁₀-alkyl, C₂-C₁₀-alkenyl, substituted or unsubstituted aryl, hetaryl, OR⁴ or NR⁴R⁵ and where the radicals R¹, R² and R³ are always different,
R⁴ hydrogen, substituted or unsubstituted, branched or unbranched C₁-C₁₀-alkyl, C₂-C₁₀-alkenyl, C₁-C₁₀-alkylcarbonyl, C₂-C₁₀-alkenylcarbonyl, aryl, arylcarbonyl, hetaryl or hetarylcarbonyl,
R⁵ hydrogen, substituted or unsubstituted, branched or unbranched C₁-C₁₀-alkyl, C₂-C₁₀-alkenyl, aryl or hetaryl.

9. The process according to claim 8, wherein one of the substituents R¹, R² or R³ is OR⁴.

10. The process according to claim 8 or 9, wherein one of the substituents R¹, R² or R³ is aryl.

11. The process according to any of claims 8 to 10, wherein the process is carried out in an aqueous reaction solution at a pH between 4 and 11.

12. The process according to any of claims 8 to 11, wherein from 0.01 to 10% by weight of nitrile or from 0.01 to 10% by weight of a corresponding aldehyde or ketone and from 0.01 to 10% by weight of hydrocyanic acid are reacted in the process.

13. The process according to any of claims 8 to 12, wherein the process is carried out at a temperature between 0°C and 80°C.

14. The process according to any of claims 8 to 13, wherein the chiral carboxylic acid is isolated from the reaction solution in yields of from 60 to 100% by extraction or crystallization or extraction and crystallization.

15. The process according to any of claims 8 to 14, wherein the chiral carboxylic acid has an optical purity of at least 90%ee.

## Revendications

1. Séquence isolée d'acide nucléique qui code pour un polypeptide ayant une activité de nitrilase, choisie dans le groupe constitué des suivantes :
a) une séquence d'acide nucléique avec la séquence représentée dans la SEQ ID n° 1,
b) des séquences d'acides nucléiques qui dérivent de la séquence d'acide nucléique représentée dans la SEQ ID n° 1 comme résultat du code génétique dégénéré,
c) des homologues de la séquence d'acide nucléique représentée dans la SEQ ID n° 1, qui codent pour des polypeptides qui présentent au moins 98% d'homologie sur la totalité du domaine de séquence avec la SEQ ID n° 2 sans que l'action enzymatique des polypeptides ne soit réduite.

2. Séquence d'acide aminé codée par une séquence d'acide nucléique selon la revendication 1.

3. Séquence d'acide aminé selon la revendication 2 codée par la séquence représentée dans la SEQ ID n° 1.

4. Construction d'acide nucléique contenant une séquence d'acide nucléique selon la revendication 1, dans laquelle la séquence d'acide nucléique est liée à un ou plusieurs signaux de régulation.

5. Vecteur contenant une séquence d'acide nucléique selon la revendication 1 ou une construction d'acide nucléique selon la revendication 4.

6. Microorganisme contenant au moins une séquence d'acide nucléique insérée selon la revendication 1 ou au moins une construction d'acide nucléique insérée selon la revendication 4.

7. Microorganisme selon la revendication 6, le microorganisme étant une bactérie des souches Escherichia, Pseudomonas ou Alcaligenes.

8. Procédé de fabrication d'acides carboxyliques chiraux de formule générale I : 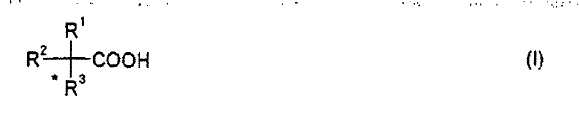 **caractérisé en ce que** l'on fait réagir des nitriles racémiques de formule générale II : 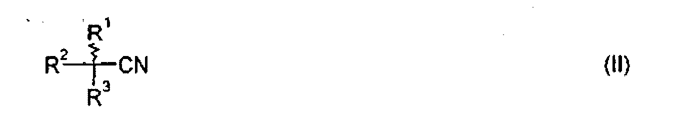 en présence d'une séquence d'acide aminé selon la revendication 2 ou 3 ou d'un microorganisme en cours de croissance, au repos ou digéré selon la revendication 6 ou 7 et dans lequel on fait réagir au moins 25 mmoles de nitrile/h par mg de protéine ou 25 mmoles de nitrile/h par g de poids sec pour former des acides carboxyliques chiraux, dans lequel les substituants et les variables des formules I et II ont la signification suivante :
* désigne un centre optiquement actif,
R¹, R², R³ représentent, indépendamment les uns des autres, de l'hydrogène, un groupement substitué ou non, ramifié ou non de type alkyle en C₁-C₁₀ et alcényle en C₂-C₁₀, un groupement substitué ou non de type aryle, hétaryle, OR⁴ ou NR⁴R⁵, et dans lequel les radicaux R¹, R² et R³ sont toujours différents,
R⁴ est de l'hydrogène, un groupement substitué ou non, ramifié ou non de type alkyle en C₁-C₁₀, alcényle en C₂-C₁₀, alkylcarbonyle en C₁-C₁₀, alcénylcarbonyle en C₁-C₁₀, aryle, arylcarbonyle, hétaryle ou hétaryl-carbonyle,
R⁵ est de l'hydrogène, un groupement substitué ou non, ramifié ou non de type alkyle en C₁-C₁₀, alcényle en C₂-C₁₀, aryle ou hétaryle.

9. Procédé selon la revendication 8, **caractérisé en ce qu'**un des substituants R¹, R² ou R³ représente OR⁴.

10. Procédé selon la revendication 8 ou 9, **caractérisé en ce que** l'un des substituants R¹, R² ou R³ représente un groupement aryle.

11. Procédé selon les revendications 8 à 10, **caractérisé en ce que** le procédé est réalisé en solution réactionnelle aqueuse à pH entre 4 et 11.

12. Procédé selon les revendications 8 à 11, **caractérisé en ce que**, dans le procédé, on fait réagir 0,01 à 10% en poids de nitrile ou 0,01 à 10% en poids d'un aldéhyde ou d'une cétone correspondant(e) et 0,01 à 10% en poids d'acide cyanhydrique.

13. Procédé selon les revendications 8 à 12, **caractérisé en ce que** le procédé est réalisé à une température de 0°C à 80°C.

14. Procédé selon les revendications 8 à 13, **caractérisé en ce que** l'acide carboxylique chiral est obtenu par extraction ou cristallisation ou extraction et cristallisation en rendements de 60 à 100% à partir de la solution réactionnelle.

15. Procédé selon les revendications 8 à 14, **caractérisé en ce que** l'acide carboxylique chiral possède une pureté optique d'au moins 90%ee.
